# EUROPEAN PATENT SPECIFICATION

(11) **EP 4 387 831 B1**
(45) Date of publication and mention of the grant of the patent: **02.04.2025**
(21) Application number: 22768265.5
(22) Date of filing: 12.08.2022
(51) Int. Cl.: B29C 64/118, B29C 64/336, B33Y 10/00, B33Y 40/00, B33Y 70/10, B33Y 80/00, A61L 9/20

(54) **3D PRINTED REFLECTORS FOR DISINFECTION LIGHTING**
3D-GEDRUCKTE REFLEKTOREN ZUR DESINFEKTION VON BELEUCHTUNGEN
RÉFLECTEURS IMPRIMÉS EN 3D POUR ÉCLAIRAGE DE DÉSINFECTION

(30) Priority: 17.08.2021 EP 21191581
(43) Date of publication of application: 26.06.2024
(73) Proprietor: Signify Holding B.V., 5656 AE Eindhoven (NL)
(72) Inventor: VAN BOMMEL, Ties, 5656 AE Eindhoven (NL); HIKMET, Rifat, Ata, Mustafa, 5656 AE Eindhoven (NL); VAN OS, Jacobus, Petrus, Johannes, 5656 AE Eindhoven (NL)
(74) Representative: Verweij, Petronella Daniëlle
(86) International application number: PCT/EP2022/072694
(87) International publication number: WO 2023/020970

(56) References cited:
- WO-A1-2018/162268
- WO-A1-2020/205565
- WO-A1-2021/104920
- QUILL TYLER ET AL: "Ultraviolet Reflectance of Microporous PTFE", 30 April 2020 (2020-04-30), pages 1 - 12, XP055876685, Retrieved from the Internet <URL:https://www.porex.com/wp-content/uploads/2020/04/Ultraviolet-Reflectance-of-Microporous-PTFE.pdf> [retrieved on 20220106]

## Description

### FIELD OF THE INVENTION

The invention relates to a method for manufacturing a 3D (printed) item. The invention also relates to the 3D (printed) item, such as obtainable with such method. Further, the invention relates to a lighting device including such 3D (printed) item.

### BACKGROUND OF THE INVENTION

Reflective materials and reflectors for light emitting diodes are known in the art. US10105460, for instance, describes a structure comprising: an ultraviolet (UV) source comprising a semiconductor device comprising an active layer disposed between an n-type region and a p-type region, wherein the active layer emits radiation having a peak wavelength in a UV range; a reflector cup disposed around the UV source; and an optically transmissive cover disposed over the reflector cup, the optically transmissive cover comprising an angled sidewall, wherein the angled sidewall of the optically transmissive cover does not make contact with the reflector cup. The reflector cup is disposed around UVLED. Reflector cup may be any suitable structure. In some embodiments, reflector cup is a hollow structure that is attached, for example, to the mount. The inner surfaces of reflector cup are reflective to UV radiation. For example, reflector cup may be fabricated from a reflective material, such as aluminum, formed by any suitable technique including, for example, machining and polishing the reflective surfaces. Alternatively, reflector cup may be fabricated from a nonreflective material that is coated on the inside with a reflective layer. Suitable examples include plastic formed by, for example, molding, injection molding, 3-D printing, or any other suitable technique. Examples of suitable reflective coatings include metals, silver, aluminum, Teflon, polytetrafluoroethylene (PTFE), barium sulfate, oxides, oxides of silicon including SiO₂, oxides of yttrium, oxides of hafnium, a multilayer stack, a distributed Bragg reflector, and combinations thereof. The reflective inner surface of reflector cup may have any suitable finish including a mirror finish, a diffusely reflective or "orange peel" finish, or a faceted finish (the facets may have a mirror finish or diffusely reflective finish).

WO-2021/104920 discloses a method for producing a 3D item by means of fused deposition modelling using a core-shell 3D printable material having (i) a core comprising core material and (ii) a shell comprising shell material. The core material comprises a core thermoplastic material and a core additive material. The shell material comprises a shell thermoplastic material and shell particles. The shell material is light transmissive for one or more wavelengths in the visible wavelength range. The shell particles comprise specularly reflective particles. The core additive material comprises one or more of diffuse reflective particles, white particles, black particles, colored particles, and dye molecules. The core material and shell material differ in one or more optical properties selected from the group of color, reflectivity, type of reflectivity, and absorption of light.

WO-2020/205565 discloses the preamble of claims 1 and 10, in particular an acrylic copolymer composition useful for 3-D printing. The composition can be formed into a uniform filament and 3-D printed using material extrusion additive manufacturing. The acrylic copolymer has a low glass transition temperature, providing the proper stiffness for good 3-D printing.

### SUMMARY OF THE INVENTION

UV light has been used for disinfection for over 100 years. Wavelengths between about 190 nm and 300 nm may be strongly absorbed by nucleic acids, which may result in defects in an organism's genome. This may be desired for inactivating (killing), bacteria and viruses, but may also have undesired side effects for humans. Therefore, the selection of wavelength of radiation, intensity of radiation and duration of irradiation may be limited in environments where people may reside such as offices, public transport, cinema's, restaurants, shops, etc., thus limiting the disinfection capacity. Especially in such environments, additional measures of disinfection may be advantageous to prevent the spread of bacteria and viruses such as influenza or novel (corona) viruses like COVID-19, SARS and MERS.

It appears desirable to produce systems, that provide alternative ways for air treatment, such as disinfection. Further, existing systems for disinfection may not easily be implemented in existing infrastructure, such as in existing buildings like offices, hospitality areas, etc. and/or may not easily be able to serve larger spaces. This may again increase the risk of contamination. Further, incorporation in HVAC systems may not lead to desirable effects and appears to be relatively complex. Further, existing systems may not be efficient, or may be relatively bulky, and may also not easily be incorporated in functional devices, such as e.g. luminaires. Other disinfection systems may use one or more anti-microbial and/or anti-viral means to disinfect a space or an object. Examples of such means may be chemical agents which may raise concerns. For instance, the chemical agents may also be harmful for people and pets. The disinfecting light may especially comprise ultraviolet (UV) radiation (and/or optionally violet radiation), i.e., the light may comprise a wavelength selected from the ultraviolet wavelength range (and/or optionally the violet wavelength range). However, other wavelengths are herein not excluded. The ultraviolet wavelength range is defined as light in a wavelength range from 100 to 380 nm and can be divided into different types of UV light / UV wavelength ranges (Table 1). Different UV wavelengths of radiation may have different properties and thus may have different compatibility with human presence and may have different effects when used for disinfection (Table 1).

**Table 1: Properties of different types of UV, violet, and NIR wavelength light**

| Name | Short name | Wavelength (nm) | (Relative) sterilization effectiveness | | Safe Radiation | Vitamin D generation | Ozone generation |
|---|---|---|---|---|---|---|---|
| | | | Bacteria | Viruses | | | |
| Near Infrared (deep red) | NIR | 780-950 | + | +/- | +++ | | |
| Violet | V | 380-420 | +/- | - | + | | |
| Ultraviolet A | UV-A | 315-380 | + | - | + | | |
| Ultraviolet B | UV-B | 280-315 | + | +/- | +/- | + | |
| Near ultraviolet C | Near UV-C | 230-280 | ++ | ++ | - | | |
| Far ultraviolet C | Far UV-C | 190-230 | +++ | +++ | + | | +/- |
| Extreme ultraviolet C | Extreme UV-C | 100-190 | +++ | +++ | - | | + |

Each UV type / wavelength range may have different benefits and/or drawbacks. Relevant aspects may be (relative) sterilization effectiveness, safety (regarding radiation), and ozone production (as result of its radiation). Depending on an application a specific type of UV light or a specific combination of UV light types may be selected and provides superior performance over other types of UV light. UV-A may be (relatively) safe and may inactivate (kill) bacteria, but may be less effective in inactivating (killing) viruses. UV-B may be (relatively) safe when a low dose (i.e. low exposure time and/or low intensity) is used, may inactivate (kill) bacteria, and may be moderately effective in inactivating (killing) viruses. UV-B may also have the additional benefit that it can be used effectively in the production of vitamin D in a skin of a person or animal. Near UV-C may be relatively unsafe, but may effectively inactivating, especially kill bacteria and viruses. Far UV-C may also be effective in inactivating (killing) bacteria and viruses, but may be (relatively to other UV-C wavelength ranges) (rather) safe. Far-UV C light may generate some ozone which may be harmful for human beings and animals. Extreme UV-C may also be effective in inactivating (killing) bacteria and viruses, but may be relatively unsafe. Extreme UV-C may generate ozone which may be undesired when exposed to human beings or animals. In some application ozone may be desired and may contribute to disinfection, but then its shielding from humans and animals may be desired. Hence, in the table "+" for ozone production especially implies that ozone is produced which may be useful for disinfection applications, but may be harmful for humans / animals when they are exposed to it. Hence, in many applications this "+" may actually be undesired while in others, it may be desired. The types of light indicated in above table may in embodiments be used to sanitize air and/or surfaces.

The terms "inactivating" and "killing" with respect to a virus may herein especially refer to damaging the virus in such a way that the virus can no longer infect and/or reproduce in a host cell, i.e., the virus may be (essentially) harmless after inactivation or killing.

Hence, The light may comprise a wavelength in the UV-A range. The light may comprise a wavelength in the UV-B range. The light may comprise a wavelength in the Near UV-C range. The light may comprise a wavelength in the Far UV-C range. The light may comprise a wavelength in the extreme UV-C range. The Near UV-C, the Far UV-C and the extreme UV-C ranges may herein also collectively be referred to as the UV-C range. Hence, The light may comprise a wavelength in the UV-C range. In other embodiments, the light may comprise violet radiation.

Standard optical elements, like reflectors, may not easily be made free-form. Further, standard materials used for optical elements, like reflectors, may not always have the desirable optical properties. When using materials that have desirable optical properties, such materials may be more difficult to process or may not easily be made free-form.

Hence, it is an aspect of the invention to provide an alternative 3D printing method and/or 3D (printed) item which preferably further at least partly obviate(s) one or more of above-described drawbacks. The present invention may have as object to overcome or ameliorate at least one of the disadvantages of the prior art, or to provide a useful alternative.

Especially, it is herein proposed to use a 3D printing solution, especially fused deposition modelling, to create optical elements, especially reflectors. Herein, amongst others solutions are provided to produce substantially free-form items, that may be suitable for reflection of UV radiation, using 3D printing, especially fused deposition modelling (though other options are herein not excluded).

Within the next 10-20 years, digital fabrication will increasingly transform the nature of global manufacturing. One of the aspects of digital fabrication is 3D printing. Currently, many different techniques have been developed in order to produce various 3D printed objects using various materials such as ceramics, metals and polymers. 3D printing can also be used in producing molds which can then be used for replicating objects.

For the purpose of making molds, the use of polyjet technique has been suggested. This technique makes use of layer by layer deposition of photo-polymerisable material which is cured after each deposition to form a solid structure. While this technique produces smooth surfaces the photo curable materials are not very stable, and they also have relatively low thermal conductivity to be useful for injection molding applications.

The most widely used additive manufacturing technology is the process known as Fused Deposition Modeling (FDM). Fused deposition modeling (FDM) is an additive manufacturing technology commonly used for modeling, prototyping, and production applications. FDM works on an "additive" principle by laying down material in layers; a plastic filament or metal wire is unwound from a coil and supplies material to produce a part. Possibly, (for thermoplastics for example) the filament is melted and extruded before being laid down. FDM is a rapid prototyping technology. Other terms for FDM are "fused filament fabrication" (FFF) or "filament 3D printing" (FDP), which are considered to be equivalent to FDM. In general, FDM printers use a thermoplastic filament, which is heated to its melting point (melting temperature) and then extruded, layer by layer, (or in fact filament after filament) to create a three-dimensional object. FDM printers are relatively fast, low cost and can be used for printing complicated 3D objects. Such printers are used in printing various shapes using various polymers. The technique is also being further developed in the production of LED luminaires and lighting solutions. Instead of a filament, 3D printable pallets may be used, which may be fed to the printer nozzle, and may leave the printer nozzle (as extrudate) to be deposited and become a 3D printed layer.

Hence, in a first aspect the invention provides a method for producing a 3D item by means of fused deposition modelling (especially with a FDM printer), wherein the method may comprise a 3D printing stage. Especially, the 3D printing stage may comprise a reflective material deposition stage. The reflective material deposition stage may comprise: providing 3D printable material and depositing the 3D printable material. The 3D printable material may comprise (i) a polymeric matrix material and a reflective material. Especially, the polymeric matrix material may be transmissive for UV radiation. Further, especially the polymeric matrix material may comprise thermoplastic material. Further, especially the reflective material may be reflective for the UV radiation. Especially, the reflective material may at least partly be enclosed by the polymeric matrix material. The reflective material may comprise a first fluoropolymer. Especially, by depositing the 3D printable material the 3D item may be provided. The 3D item may (thus) comprise 3D printed material comprising the matrix material and the reflective material. Hence, in embodiments the invention provides a method for producing a 3D item by means of fused deposition modelling, the method comprising a 3D printing stage, wherein the 3D printing stage comprises a reflective material deposition stage, wherein the reflective material deposition stage comprises: (A) providing 3D printable material comprising (i) a polymeric matrix material that is transmissive for UV radiation, especially wherein the polymeric matrix material comprises thermoplastic material, and (ii) a reflective material that is reflective for the UV radiation and that is at least partly enclosed by the polymeric matrix material; wherein the reflective material comprises a first fluoropolymer; and (B) depositing the 3D printable material, to provide the 3D item comprising 3D printed material comprising the matrix material and the reflective material. In yet a further aspect (or in yet further embodiments), the invention provides a method for producing a 3D item by means of fused deposition modelling, the method comprising (i) a 3D printing stage comprising layer-wise depositing (an extrudate comprising) 3D printable material, to provide the 3D item comprising 3D printed material (on a receiver item), wherein the 3D item may comprise a plurality of layers of 3D printed material, wherein the 3D printing stage comprises a reflective material deposition stage, wherein the reflective material deposition stage comprises: (A) providing 3D printable material comprising (i) a polymeric matrix material that is transmissive for UV radiation, especially wherein the polymeric matrix material comprises thermoplastic material, and (ii) a reflective material that is reflective for the UV radiation and that is at least partly enclosed by the polymeric matrix material; wherein the reflective material comprises a first fluoropolymer; and (B) depositing the 3D printable material, to provide the 3D item comprising 3D printed material comprising the matrix material and the reflective material.

With such method, it may be possible to produce optical elements, especially reflectors, which may be substantially free-form items. Further, with such method it may be possible to provide (free-form) items that may be reflective of UV radiation. Further, the invention provides a solution to the use of highly UV reflective materials that may be difficult to process with FDM. Further, the use of at least two different materials may also allow choosing good combinations of mechanical and/or optical properties, and optionally also other properties. The reflective material may be used for its reflectivity and the matrix material may be used to host the reflective material and to provide mechanical strength to the 3D item.

As indicated above, the 3D printing stage may comprise a reflective material deposition stage. Hence, the 3D printing stage may in embodiments also comprise other stages, such as a stage wherein 3D printable material is deposited that does not comprise the reflective material and/or does not comprise the polymeric matrix material. For instance, part of the 3D item may comprise 3D printed material essentially only consisting of the polymeric matrix material without reflective material. Hence, the material composition of different 3D printed layers may be different. Even, in specific embodiments the material composition of a 3D printed layer may vary over e.g. the layer length. Hence, if desirable, the composition of the 3D printed layers may vary over the 3D item. For instance, in the case of a hollow reflector, the surface of the hollow reflector that should be reflective may be produced during the reflective material deposition stage whereas an external part, or support elements for the surface of the hollow reflector that should be reflective, may be produced during a 3D printing stage where not necessarily reflective material is used.

As indicated above, the reflective material deposition stage may comprise: (i) providing 3D printable material and (ii) depositing the 3D printable material. When the 3D printable material is deposited, it is indicated as 3D printed material.

Hence, the reflective material deposition stage may comprise: providing 3D printable material comprising (i) a polymeric matrix material and (ii) a reflective material.

Especially, in embodiments the polymeric matrix material is transmissive for UV radiation. Hence, at least part of UV radiation having one or more wavelengths selected from the range of 100-380 nm, even more especially at least selected from the range of 190-380 nm may be transmitted by the polymeric matrix material. The term "matrix material" may especially be applied to indicate that the polymeric matrix material may at least partly enclose the reflective material (see also below). Especially, the polymeric matrix material may comprise thermoplastic material. The thermoplastic material may relatively easily be 3D printed with a fused deposition modelling printer. The thermoplastic material may be deformed in the printer nozzle and may especially allow providing 3D printed material layers.

Further, especially in embodiments the reflective material may be reflective for the UV radiation. Hence, at least part of UV radiation having one or more wavelengths selected from the range of 100-380 nm, even more especially at least selected from the range of 190-380 nm, may be reflected by the polymeric matrix material.

Especially, assuming perpendicular irradiation, the reflection of the reflective material for the UV radiation may be larger than the transmission and absorption of the UV radiation by the reflective material. Likewise, assuming perpendicular irradiation, especially the transmission of the UV radiation by the polymeric matrix material may be larger than the reflection or absorption of the UV radiation by the polymeric matrix material. Note that these definitions do not necessarily imply that (only) perpendicular irradiation is applied; this formulation is used to define e.g. reflection or transmission.

The term "reflective material" may also refer to a combination of two or more different materials; together they may form the reflective material.

The reflective material may comprise a first fluoropolymer, such as e.g. selected from the group of poly(tetrafluoroethylene) (PTFE), poly(tetrafluoroethylene-co-hexafluoropropylene) (FEP), poly(ethylene-alt-tetrafluoroethylene) (ETFE), poly(chlorotrifluoroethylene) (PCTFE), poly(vinylidene fluoride )(PVDF), poly(tetrafluoroethylene-co-perfluoropropyl vinyl ether) (PFA), poly(vinylidene fluoride-co-hexafluoropropylene) (PVDF-co-HFP), and poly(vinyl fluoride) (PVF).

Fluorothermoplastics typically are copolymers of tetrafluoroethylene (TFE) with one or more other perfluorinated, partially fluorinated or non-fluorinated comonomers. Copolymers of TFE and perfluorinated alkyl or allyl ethers are known in the art as PFA's (perfluorinated alkoxy polymers). Copolymers of TFE and hexafluoropropylene (HFP) with or without other perfluorinated comonomers are known in the art as FEP's (fluorinated ethylene propylene). Copolymers of TFE, HFP and vinylidenefluoride (VDF) are known in the art as THV. Other types of melt-processable fluoropolymers are based on vinylidenefluoride homo- or copolymers, known in the art as PVDF. Other class of fluorothermoplastic is fluorinated ethylenic-cyclo oxyaliphatic substituted ethylenic copolymer is a family of amorphous fluoropolymers based on copolymers of 2,2-bistrifluoromethyl-4,5-difluoro-1,3-dioxole (PDD. Some fluoropolymers are known by their commercial names such as Teflon, Aflas, Vitom, Fluorel, Cytop, Karlez. Especially such fluoropolymers may be used as first fluoropolymer.

Especially, in embodiments the first fluoropolymer may comprise a microporous polytetrafluoroethylene. The term "first fluoropolymer" may also refer to a combination of two or more different first fluoropolymers.

Especially good results may be obtained when the first fluoropolymer is microporous. Then reflectivity for UV radiation may especially be relatively high. Hence, in embodiments the first fluoropolymer may comprise a (first) microporous fluoropolymer.

The first fluoropolymer may be in the form of continuous or cut ribbons and or fibers. The fibers may form agglomerates. In that case, the air gaps within the agglomerates may lead to light scattering.

It is also possible to use polycrystalline fluoropolymer as first fluoropolymer. Polycrystalline fluoropolymer may also be light scattering as a result of microcrystals.

The reflective material, especially the first polymer, may have a porosity, such as due to air gaps between the fibers within the agglomerates.

Especially, in embodiments the first fluoropolymer may comprise pores having one or more dimensions selected from the range of 1-40 µm. For instance, pore sizes may be determined on the basis of mercury pressure porosimetry. More especially, in embodiments the first fluoropolymer may comprise pores having one or more dimensions selected from the range of 1-30 µm, such as selected from the range of 1-20 µm, like selected from the range of 1-10 µm.

In specific embodiments, the median pore diameter may be selected from the range of about 0.1-10 µm, such as selected from the range 0f 0.5-10 µm.

Especially, in embodiments the first fluoropolymer may have a porosity (volume void fraction) selected from the range about 20-70%, like especially selected from the range of 30-60%, more especially selected from the range of about 35-50%. A porosity of about 35-45%, such as 37-43% may give a relatively high reflection.

Further, as indicated above, especially the reflective material may at least partly be enclosed by the polymeric matrix material. For instance, this may in embodiments be the reflective material being embedded as particulate material in the polymeric matrix material and/or this may in (other) embodiments be the reflective material being enclosed as core by a shell of the polymeric matrix material; see further also below.

As indicated above and as further elucidated below, in embodiments the polymeric matrix material and (ii) a reflective material may be provided, such as to a nozzle of a 3D printer (especially a FDM printer). This material may be deposited, such as on a substrate (or receiver item) (or on 3D printed material earlier printed on the substrate). Hence, the reflective material deposition stage may further comprise: depositing the 3D printable material, especially on a substrate (or receiver item) (or on 3D printed material earlier printed on the substrate). In this way, the method may provide the 3D item comprising 3D printed material comprising the matrix material and the reflective material. Hence, the 3D printable material has in this way been printed and essentially thereby become 3D printed material.

As indicated above, the matrix material may be transmissive for UV radiation, or at least to one or more wavelengths selected from the range of 100-380 nm, more especially 190-380 nm. The matrix material may thus have a relatively low reflectivity, and may be used as a host for the reflective material.

The matrix material may comprise a polyolefin such as polyethylene. Other options may include e.g. an acrylic polymer, such as polymethylacrylate (PMA), Perspex, or polymethylmethacrylate, PMMA. The term "matrix material" may also refer to a combination of two or more different materials; together may form the matrix material. The matrix material may (also) be an amorphous fluoropolymer or a fluoropolymer with a melting temperature below 300 °C (so that it can be used in FDM printing). When the matrix material may comprise a fluoropolymer, the fluoropolymer is indicated as "second fluoropolymer.

First fluoropolymers, such as e.g. polytetrafluoroethylene, may be relatively difficult to use in fused deposition modelling. Such polymers may have a high melting temperature and/or may have a high glass temperature, or may substantially crystalline. Compared to e.g. polylactic acid or PMMA, the melting temperature of polytetrafluoroethylene is substantially higher. Hence, polytetrafluoroethylene may be a material that is difficult to 3D print with a FDM process. Therefore, solutions may have to be provided to have the advantages of the UV reflectivity of the first fluoropolymers, such as e.g. polytetrafluoroethylene, while still being able to 3D print such first fluoropolymers.

Basically there may be two options. In a first option, it is substantially not attempted to extrude the first fluoropolymer, but the first fluoropolymer is simply fed as fiber through the printer nozzle, e.g. in a core part of the nozzle. This can be done with a core-shell nozzle, but also with a nozzle with a single opening. The fiber may be guided through the nozzle and the 3D printable matrix material may substantially surround the first fluoropolymer (fiber). In a second option, the first fluoropolymer may be made more 3D printable, substantially without losing its reflectivity, by embedding first fluoropolymer particles in another matrix.

Hence, in embodiments the reflective material deposition stage may comprise (a) guiding a fiber (comprising the first fluoropolymer) through a 3D printer nozzle, while also (b) providing the polymeric matrix material to the 3D printer nozzle to provide core-shell 3D printed material. In such embodiments, the fiber may be guided through the 3D printer nozzle without melting and/or without substantial softening of the first fluoropolymer (e.g. by surpassing a possible melting temperature of the first fluoropolymer). This may be executed with a core-shell nozzle, or with a nozzle with a single opening; the former may allow an easier control of the reflective material deposition stage. Hence, in specific embodiments the fiber may comprise microporous PTFE. The fiber may essentially consist of the first fluoropolymer, such as at least 90 vol.% of the fiber consisting of first fluoropolymer. In yet other embodiments, the fiber may have a core of a different material, and a coating or cladding of first fluoropolymer.

Alternatively or additionally, in embodiments the 3D printable material may comprise particulate material comprising the reflective material, wherein the particulate material may be embedded in the matrix material. The particles may e.g. have dimensions selected from the range of 1 µm - 1 mm, such as especially selected from the range of 1-500 µm. In specific embodiments, one or more dimensions may be selected from the range of 2-100 µm. Especially, the particles may be smaller than the smallest dimension of a 3D printer nozzle opening through which the particles have to be transported. Further, especially the particles may have dimensions smaller than a height and/or a width of a 3D printed layer (to be printed) wherein the particles will be available, such as equal to or less than 80% of the height and/or a width of a 3D printed layer (to be printed) wherein the particles will be available.

The particulate material may comprise substantially spherical particles or agglomerates of particles with air trapped between the particles. Alternatively or additionally, the particulate material may comprise non-spherical particles. The particulate material may comprise flake-like particles. The particulate material may comprise white or metallic. The particulate material may comprise coated with a coating, such as a metallic coating. Further, the particles may comprise one or more type of particles, wherein the particles may differ in size distributions, like a bimodal size distribution, and/or in type. For instance, the particles may comprise white particles and reflective particles. The particulate material may comprise a second reflective material selected from the group of BaSO₄ particles, TiO₂ particles, Al₂O₃ particles, silver particles, aluminum particles, and reflective flakes. Using different particles may provide a good reflection over a broader wavelength range. Reflective flakes may be non-metal flakes with a reflective coating. The coating may be a metal coating, like a silver coating or an aluminum coating. However, the flakes may also be metal flakes (and may then also be considers as metal reflective particles). Flakes may be embodiments of platelets. Particles may in embodiments have dimensions (length, width, height, diameter) selected from the range of 50-200 µm, though other sizes may also be possible. Here, the particle size may especially refer to a number averaged particle size. Particle sizes may be determined with methods known in the art, like one or more of optical microscopy, SEM (scanning electron microscope) and TEM (transmission electron microscopy). Dimensions may be number averaged, as known in the art. Hence, the particles may be substantially identical, but the particles may also mutually differ, such as two or more subsets of particles, wherein within the subsets the particles are substantially identical. The particles may have a unimodal particle size distribution or a polymodal size distribution.

When using particulate material, it may be available in the entire 3D printable (and 3D printed material), like throughout the entire cross-section of a filament. However, it may also be concentrated in a core part, with a higher concentration in a core of the 3D printable material and 3D printed material, than in a shell. There may essentially be no first fluoropolymer comprising material in a shell of the 3D printable material and 3D printed material. This may allow using less first fluoropolymer while still having a good reflection. Hence, in embodiments the method may comprise depositing 3D printable material comprising a core and a shell, wherein the core may comprise the reflective material and wherein the shell may comprise the matrix material. In such embodiments, the core may comprise thermoplastic material, with UV reflective particulate material embedded therein. The thermoplastic material of the core and the shell may be the same or may be different.

| **Non-limiting examples of embodiments** | | |
|---|---|---|
| **Reflective material** | **Matrix material** | **Configuration** |
| Fiber of crystalline first fluoropolymer | Thermoplastic polymer | Core reflective material - shell matrix material |
| Amorphous first fluoropolymer | Thermoplastic polymer | Core reflective material - shell matrix material |
| Amorphous first fluoropolymer + (inorganic) reflective particles | Thermoplastic polymer | Core reflective material - shell matrix material |
| Fiber of crystalline first fluoropolymer | Thermoplastic polymer comprising second fluoropolymer (different from first fluoropolymer) | Core reflective material - shell matrix material |
| Amorphous first fluoropolymer | Thermoplastic polymer comprising second fluoropolymer (different from first fluoropolymer) | Core reflective material - shell matrix material |
| Amorphous first fluoropolymer + (inorganic) reflective particles | Thermoplastic polymer comprising second fluoropolymer (different from first fluoropolymer) | Core reflective material - shell matrix material |
| Particles of (fibrous) crystalline first fluoropolymer | Thermoplastic polymer | reflective material distributed in the matrix material |
| Particles of (fibrous) amorphous first fluoropolymer | Thermoplastic polymer | reflective material distributed in the matrix material |
| Particles of (fibrous) amorphous first fluoropolymer + (inorganic) reflective particles | Thermoplastic polymer | reflective material distributed in the matrix material |
| Particles of (fibrous) crystalline first fluoropolymer | Thermoplastic polymer comprising second fluoropolymer (different from first fluoropolymer) | reflective material distributed in the matrix material |
| Particles of (fibrous) amorphous first fluoropolymer | Thermoplastic polymer comprising second fluoropolymer (different from first fluoropolymer) | reflective material distributed in the matrix material |
| Particles of (fibrous) amorphous first fluoropolymer + (inorganic) reflective particles | Thermoplastic polymer comprising second fluoropolymer (different from first fluoropolymer) | reflective material distributed in the matrix material |

Note that the fiber of first fluoropolymer or the particles of first fluoropolymer may comprise agglomerates of fluoropolymer fibers.

The 3D printable material and the 3D printed material may consist for at least 90 vol% of the reflective material and the matrix material, such as at least 95 vol.%, more especially essentially 100 vol%. The 3D printable material and the 3D printed material may consist for at least 20 vol% of the reflective material and/or for at least 20 vol% of the matrix material, more especially for at least 30 vol% of the reflective material and/or for at least 30 vol% of the matrix material. Even more especially, the 3D printable material and the 3D printed material may consist for at least 40 vol% of the reflective material and/or for at least 30 vol% of the matrix material.

In embodiments, a layer of the 3D printed material may have a width (w1) and a height (h1). Especially, the width (w1) and a height (h1) may individually be selected from the range of 100 - 5000 µm, such as especially selected from the range of 100-3000 µm. Especially, the width (w1) and a height (h1) may individually be selected from the range 0.1-10 mm, like at maximum 8 mm, such as in embodiments 0.1-3 mm. Further, in specific embodiments the shell may have a largest shell width (wₛₘ₁). Especially, in embodiment the largest shell width (wₛₘ₁) may be selected from the range of 1-25% of the width (w1), even more especially, 2-15% of the width (w1), like in embodiments selected from the range of 5-15%.

The core-shell option also allows providing better adhering layers, which adhere better to each other, while having a good reflection for UV radiation.

Layers may be stacked. In this way the 3D item may get a height (relative to the printing stage) of one or more, especially a plurality of layers stacked one on another. It is also possible to configure layers adjacent to each other. In this way, the 3D item or 3D item part, may get a thickness, which may be at least a layer thickness, such as the thickness of two or more layers. Part of at least one of the layers, such as an entire layer, may essentially consist of the matrix material and reflective material. Hence, part of at least one of the layers may comprise (other) 3D printed material. In embodiments, such part(s) of may be light absorbing, though this is not necessarily the case. Such part of at least one of the layers may comprise does thus not necessarily comprise the herein described reflective material. Such part of at least one of the layers may be available for support of the part of at least one of the layers, essentially consisting of the matrix material and reflective material. However, other functions may also possible. It may not be necessary that the entire 3D item is UV reflective.

As indicated above, the polymeric matrix material may be transmissive for UV radiation having one or more wavelengths selected from the range of 190-380 nm. Further, in embodiments the reflective material may be reflective for the UV radiation having the one or more wavelengths selected from the range of 190-380 nm. Especially, the polymeric matrix material, the reflective material, the relative amounts, the thickness of the 3D printed layer, etc. may be selected such that under perpendicular radiation with the UV radiation having the one or more wavelengths selected from the range of 190-380 nm a layer comprising the 3D printed material reflects at least 40% of the UV radiation having the one or more wavelengths selected from the range of 190-380 nm. Hence, in embodiments the polymeric matrix material may be transmissive for UV radiation having one or more wavelengths selected from the range of 190-380 nm, and wherein the reflective material is reflective for the UV radiation having the one or more wavelengths selected from the range of 190-380 nm; wherein polymeric matrix material, the reflective material, the relative amounts, the thickness of the 3D printed layer, etc. are selected such that under perpendicular radiation with the UV radiation having the one or more wavelengths selected from the range of 190-380 nm a layer comprising the 3D printed material reflects at least 40% of the UV radiation having the one or more wavelengths selected from the range of 190-380 nm. Percentage may refer to percentage of the spectral power (e.g. in Watts) of the UV radiation.

The first fluoropolymer of the reflective material may in embodiments have a melting temperature, which may be indicated as a first melting temperature T_{f1,m}. The first fluoropolymer may in specific embodiments be a thermoplastic polymer. As indicated above the polymeric matrix material may comprise a thermoplastic polymer. The thermoplastic polymer may have melting temperature, which may be indicated as second melting temperature T_{2,m}. Especially, the latter may be lower than the former. For instance, in embodiments T_{2,m}≤T_{f1,m}-10°C. Especially, T_{f1,m} ≥ 280 °C, like in embodiments T_{f1,m} ≥ 300 °C, such as in specific embodiments T_{f1,m} ≥ 320 °C. Alternatively or additionally, T_{2,m} < 280 °C, like in embodiments T_{2,m}≤ 260 °C, such as in specific embodiments T_{2,m}≤ 260 °C.

In further specific embodiments, the thermoplastic polymer of the matrix material may also be a fluoropolymer (but especially different from the first fluoropolymer of the reflective material). Hence, in specific embodiments the first fluoropolymer may comprise a first melting temperature T_{f1,m}, and the matrix material may comprise a thermoplastic polymer, such as in specific embodiments a second fluoropolymer, having a second fluoropolymer melting temperature T_{f2,m}, wherein T_{f2,m}≤T_{f1,m}-10°C. Especially, T_{f1,m} ≥ 280 °C, like in embodiments T_{f1,m} ≥ 300 °C, such as in specific embodiments T_{f1,m} ≥ 320 °C. Alternatively or additionally, T_{f2,m} < 280 °C, like in embodiments T_{f2,m}≤ 260 °C, such as in specific embodiments T_{f2,m}≤ 260°C.

Hence, the polymeric material of the matrix material may have a lower melting temperature than the polymeric material of the reflective material. Especially, the difference is at least 10°C, more especially at least 20°C, yet even more especially at least 30°C.

Yet, in further embodiments, (during operation) the nozzle temperature may be lower than the first melting temperature T_{f1,m}. Further, the thermoplastic polymer of the matrix material may have a glass transition temperature T_{f2,g}. Yet, in further embodiments, (during operation) the nozzle temperature may be at least the glass transition temperature T_{f2,g}.

Note, however, that the reflective material may in embodiments also have a glass transition temperature, e.g. when an amorphous fluoropolymer is used. Though not necessarily, the glass transition temperature of the reflective material T_{f1,g} (when having a glass transition temperature), may be smaller than the glass transition temperature T_{2,g} of the matrix material. For instance, in embodiments T_{2,g}≤T_{f1,g}-10°C may apply, such as T_{2,g}≤T_{f1,g}-20°C, such as especially T_{2,g}≤T_{f1,g}-30°C.

The method may be used to produce reflectors, like hollow reflectors. Hence, in embodiments the 3D item may comprise a hollow reflector. The method may (thus) be used to produce a collimator, such as a hollow collimator. The 3D item may be a reflective collimator, like a hollow reflective collimator.

Here below, some further embodiments are described.

In embodiments of a core-shell filament or a core-shell layer, a cross-sectional parameter of the core of a core-shell filament or a core-shell layer may be selected from the range of 0.7-0.95 times the cross-sectional parameter of the filament or layer, respectively. The cross-section parameters may be defined along coinciding lines. This may provide a relatively high reflection but also a relatively good adhesion.

The cross-sectional parameter of the core of a core-shell filament or a core-shell layer may in embodiments be at least 0.5 mm and/or the cross-sectional parameter of the filament or layer, respectively, may be at maximum 3 mm. This may provide sufficient UV reflection, but may also allow acceptable material usage. A cross-sectional dimension of the core may in embodiments be selected from the range of 1.5-2.5, such as about 1.75-2.25 mm.

The shell of the core of a core-shell filament or a core-shell layer may also comprise UV reflective particles but its volume percentage is typically less than of the core (in case of a core-shell filament or a core-shell layer wherein the core may comprise UV reflective particulate material). For instance, the volume percentage of the UV reflective particles in the shell may be 50% or less than the volume percentage of the UV reflective particles in the core.

In order to promote adhesion the FDM filament or 3D printed layer may comprise a combination of microporous PTFE particles and particles/flakes of another reflective material such as for example BaSO₄ or Al particles (see also above). This may also allow a relatively high(er) (reflective) particle loading.

The particle loading of the core of a core-shell filament or a core-shell layer may especially be at least 40% volume% (relative to the volume of the core), such as at least 50 v/v% (relative to the volume of the core), such as even at least 60 volume %. This may provide a relatively high reflection.

As indicated above, the core of a core-shell filament or a core-shell layer may comprise a 3D printable polymeric material (or 3D printed polymeric material), especially a thermoplastic material, and the shell may comprise a 3D printable polymeric material (or 3D printed polymeric material). The former may comprise UV reflective particulate material. The 3D printable polymeric material (or 3D printed polymeric material) of the core and the 3D printable polymeric material (or 3D printed polymeric material) of the shell may comprise the same polymeric material, more especially may be the same polymeric material. For instance, in such embodiments it may be possible to provide a high concentration of reflective particles in the core, while still using relatively well 3D printable material, and a relatively transparent shell.

Two layers may be printed next to each other, preferably in contact with each other, and most preferably out of phase with each other; this may improve reliability. Hence, one may stack non-reflecting and reflecting, or one may place both materials next to each other.

The UV reflective material may be porous (see also above).

In yet a further aspect, the invention provides a method for producing a 3D item by means of 3D printing, such as fused deposition modelling (FDM, with a fused deposition modelling printer), or stereolithography (SL) printing (with a stereolithographic apparatus SLA), the method comprising a 3D printing stage, wherein the 3D printing stage may comprise a reflective material deposition stage, wherein the reflective material deposition stage may comprise: (A) providing 3D printable material comprising (i) a polymeric matrix material that is transmissive for UV radiation, especially wherein the polymeric matrix material may comprise thermoplastic material, and (ii) a reflective material that is reflective for the UV radiation and that is at least partly enclosed by the polymeric matrix material; wherein the reflective material may comprise a first fluoropolymer; and (B) depositing the 3D printable material, to provide the 3D item comprising 3D printed material comprising the matrix material and the reflective material. In yet a further aspect (or in yet further embodiments), the invention provides a method for producing a 3D item by 3D printing, such as fused deposition modelling or SLA printing, the method comprising (i) a 3D printing stage comprising layer-wise depositing (an extrudate comprising) 3D printable material, to provide the 3D item comprising 3D printed material (on a receiver item), wherein the 3D item may comprise a plurality of layers of 3D printed material, wherein the 3D printing stage may comprise a reflective material deposition stage, wherein the reflective material deposition stage may comprise: (A) providing 3D printable material comprising (i) a polymeric matrix material that is transmissive for UV radiation, especially wherein the polymeric matrix material may comprise thermoplastic material, and (ii) a reflective material that is reflective for the UV radiation and that is at least partly enclosed by the polymeric matrix material; wherein the reflective material may comprise a first fluoropolymer; and (B) depositing the 3D printable material, to provide the 3D item comprising 3D printed material comprising the matrix material and the reflective material.

Stereolithography may be based on using light, such as UV radiation, to cure liquid resin into a solid object, in general one layer at a time. In this way, layer by layer a 3D item can be printed.

Here below, yet some further embodiments are described.

As indicated above, the method may comprise depositing during a printing stage 3D printable material. Herein, the term "3D printable material" refers to the material to be deposited or printed, and the term "3D printed material" refers to the material that is obtained after deposition. These materials may be essentially the same, as the 3D printable material may especially refer to the material in a printer head or extruder at elevated temperature and the 3D printed material refers to the same material, but in a later stage when deposited. The 3D printable material is printed as a filament and deposited as such. The 3D printable material may be provided as filament or may be formed into a filament. Hence, whatever starting materials are applied, a filament comprising 3D printable material is provided by the printer head and 3D printed. The term "extrudate" may be used to define the 3D printable material downstream of the printer head, but not yet deposited. The latter is indicated as "3D printed material". In fact, the extrudate may comprise 3D printable material, as the material is not yet deposited. Upon deposition of the 3D printable material or extrudate, the material is thus indicated as 3D printed material. Essentially, the materials are the same material, as the thermoplastic material upstream of the printer head, downstream of the printer head, and when deposited, is essentially the same material.

Herein, the term "3D printable material" may also be indicated as "printable material. The term "polymeric material" may in embodiments refer to a blend of different polymers, but may in embodiments also refer to essentially a single polymer type with different polymer chain lengths. Hence, the terms "polymeric material" or "polymer" may refer to a single type of polymers but may also refer to a plurality of different polymers. The term "printable material" may refer to a single type of printable material but may also refer to a plurality of different printable materials. The term "printed material" may refer to a single type of printed material but may also refer to a plurality of different printed materials.

Hence, the term "3D printable material" may also refer to a combination of two or more materials. In general, these (polymeric) materials have a glass transition temperature T_{g} and/or a melting temperature Tₘ. The 3D printable material will be heated by the 3D printer before it leaves the nozzle to a temperature of at least the glass transition temperature, and in general at least the melting temperature. Hence, in a specific embodiment the 3D printable material may comprise a thermoplastic polymer having a glass transition temperature (T_{g}) and /or a melting temperature (Tₘ), and the printer head action may comprise heating the 3D printable material above the glass transition and if it is a semi-crystalline polymer above the melting temperature. In yet another embodiment, the 3D printable material may comprise a (thermoplastic) polymer having a melting temperature (Tₘ), and the printer head action may comprise heating the 3D printable material to be deposited on the receiver item to a temperature of at least the melting temperature. The glass transition temperature is in general not the same thing as the melting temperature. Melting is a transition which occurs in crystalline polymers. Melting happens when the polymer chains fall out of their crystal structures, and become a disordered liquid. The glass transition is a transition which happens to amorphous polymers; that is, polymers whose chains are not arranged in ordered crystals, but are just strewn around in any fashion, even though they are in the solid state. Polymers can be amorphous, essentially having a glass transition temperature and not a melting temperature or can be (semi) crystalline, in general having both a glass transition temperature and a melting temperature, with in general the latter being larger than the former. The glass temperature may e.g. be determined with differential scanning calorimetry. The melting temperature or melting temperature can also be determined with differential scanning calorimetry.

As indicated above, the invention thus provides a method comprising providing a filament of 3D printable material and printing during a printing stage said 3D printable material on a substrate, to provide said 3D item.

Materials that may especially qualify as 3D printable materials may be selected from the group consisting of metals, glasses, thermoplastic polymers, silicones, etc. Especially, the 3D printable material may comprise a (thermoplastic) polymer selected from the group consisting of ABS (acrylonitrile butadiene styrene), Nylon (or polyamide), Acetate (or cellulose), PLA (poly lactic acid), terephthalate (such as PET polyethylene terephthalate), Acrylic (polymethylacrylate, Perspex, polymethylmethacrylate, PMMA), Polypropylene (or polypropene), Polycarbonate (PC), Polystyrene (PS), PE (such as expanded- high impact-Polythene (or polyethene), Low density (LDPE) High density (HDPE)), PVC (polyvinyl chloride) Polychloroethene, such as thermoplastic elastomer based on copolyester elastomers, polyurethane elastomers, polyamide elastomers polyolefine based elastomers, styrene based elastomers, etc.. Optionally, the 3D printable material may comprise a 3D printable material selected from the group consisting of Urea formaldehyde, Polyester resin, Epoxy resin, Melamine formaldehyde, thermoplastic elastomer, etc... Optionally, the 3D printable material may comprise a 3D printable material selected from the group consisting of a polysulfone. Elastomers, especially thermoplastic elastomers, are especially interesting as they are flexible and may help obtaining relatively more flexible filaments comprising the thermally conductive material. A thermoplastic elastomer may comprise one or more of styrenic block copolymers (TPS (TPE-s)), thermoplastic polyolefin elastomers (TPO (TPE-o)), thermoplastic vulcanizates (TPV (TPE-v or TPV)), thermoplastic polyurethanes (TPU (TPU)), thermoplastic copolyesters (TPC (TPE-E)), and thermoplastic polyamides (TPA (TPE-A)).

Suitable thermoplastic materials, such as also mentioned in WO2017/040893, may include one or more of polyacetals (e.g., polyoxyethylene and polyoxymethylene), poly(C₁₋₆ alkyl)acrylates, polyacrylamides, polyamides, (e.g., aliphatic polyamides, polyphthalamides, and polyaramides), polyamideimides, polyanhydrides, polyarylates, polyarylene ethers (e.g., polyphenylene ethers), polyarylene sulfides (e.g., polyphenylene sulfides), polyarylsulfones (e.g., polyphenylene sulfones), polybenzothiazoles, polybenzoxazoles, polycarbonates (including polycarbonate copolymers such as polycarbonate-siloxanes, polycarbonate-esters, and polycarbonate-ester-siloxanes), polyesters (e.g., polycarbonates, polyethylene terephthalates, polyethylene naphtholates, polybutylene terephthalates, polyarylates), and polyester copolymers such as polyester-ethers), polyetheretherketones, polyetherimides (including copolymers such as polyetherimide-siloxane copolymers), polyetherketoneketones, polyetherketones, polyethersulfones, polyimides (including copolymers such as polyimide- siloxane copolymers), poly(C₁₋₆ alkyl)methacrylates, polymethacrylamides, polynorbornenes (including copolymers containing norbornenyl units), polyolefins (e.g., polyethylenes, polypropylenes, polytetrafluoroethylenes, and their copolymers, for example ethylene- alpha- olefin copolymers), polyoxadiazoles, polyoxymethylenes, polyphthalides, polysilazanes, polysiloxanes, polystyrenes (including copolymers such as acrylonitrile-butadiene-styrene (ABS) and methyl methacrylate-butadiene-styrene (MBS)), polysulfides, polysulfonamides, polysulfonates, polysulfones, polythioesters, polytriazines, polyureas, polyurethanes, polyvinyl alcohols, polyvinyl esters, polyvinyl ethers, polyvinyl halides, polyvinyl ketones, polyvinyl thioethers, polyvinylidene fluorides, or the like, or a combination comprising at least one of the foregoing thermoplastic polymers. Embodiments of polyamides may include, but are not limited to, synthetic linear polyamides, e.g., Nylon-6,6; Nylon-6,9; Nylon-6,10; Nylon-6,12; Nylon-11; Nylon-12 and Nylon-4,6, preferably Nylon 6 and Nylon 6,6, or a combination comprising at least one of the foregoing. Polyurethanes that can be used include aliphatic, cycloaliphatic, aromatic, and polycyclic polyurethanes, including those described above. Also useful are poly(C₁₋₆ alkyl)acrylates and poly(C₁₋₆ alkyl)methacrylates, which include, for instance, polymers of methyl acrylate, ethyl acrylate, acrylamide, methacrylic acid, methyl methacrylate, n-butyl acrylate, and ethyl acrylate, etc. In embodiments, a polyolefine may include one or more of polyethylene, polypropylene, polybutylene, polymethylpentene (and co-polymers thereof), polynorbornene (and co-polymers thereof), poly 1-butene, poly(3-methylbutene), poly(4-methylpentene) and copolymers of ethylene with propylene, 1-butene, 1-hexene, 1-octene, 1-decene, 4-methyl-l-pentene and 1-octadecene.

The 3D printable material (and the 3D printed material) comprise one or more of polycarbonate (PC), polyethylene (PE), high-density polyethylene (HDPE), polypropylene (PP), polyoxymethylene (POM), polyethylene naphthalate (PEN), styrene-acrylonitrile resin (SAN), polysulfone (PSU), polyphenylene sulfide (PPS), and semi-crystalline polytethylene terephthalate (PET), acrylonitrile butadiene styrene (ABS), poly(methyl methacrylate) (PMMA), polystyrene (PS), and styrene acrylic copolymers (SMMA).

The term 3D printable material is further also elucidated below, but especially refers to a thermoplastic material, optionally including additives, to a volume percentage of at maximum about 60%, especially at maximum about 30 vol.%, such as at maximum 20 vol.% (of the additives relative to the total volume of the thermoplastic material and additives).

The printable material may thus in embodiments comprise two phases. The printable material may comprise a phase of printable polymeric material, especially thermoplastic material (see also below), which phase is especially an essentially continuous phase. In this continuous phase of thermoplastic material polymer additives such as one or more of antioxidant, heat stabilizer, light stabilizer, ultraviolet light stabilizer, ultraviolet light absorbing additive, near infrared light absorbing additive, infrared light absorbing additive, plasticizer, lubricant, release agent, antistatic agent, anti-fog agent, antimicrobial agent, colorant, laser marking additive, surface effect additive, radiation stabilizer, flame retardant, anti-drip agent may be present. The additive may have useful properties selected from optical properties, mechanical properties, electrical properties, thermal properties, and mechanical properties (see also above).

The printable material in embodiments may comprise particulate material, i.e. particles embedded in the printable polymeric material, which particles form a substantially discontinuous phase. The number of particles in the total mixture is especially not larger than 60 vol.%, relative to the total volume of the printable material (including the (anisotropically conductive) particles) especially in applications for reducing thermal expansion coefficient. For optical and surface related effect number of particles in the total mixture is equal to or less than 20 vol.%, such as up to 10 vol.%, relative to the total volume of the printable material (including the particles). Hence, the 3D printable material especially refers to a continuous phase of essentially thermoplastic material, wherein other materials, such as particles, may be embedded. Likewise, the 3D printed material especially refers to a continuous phase of essentially thermoplastic material, wherein other materials, such as particles, are embedded. The particles may comprise one or more additives as defined above. Hence, in embodiments the 3D printable materials may comprise particulate additives.

The printable material is printed on a receiver item. Especially, the receiver item can be the building platform or can be comprised by the building platform. The receiver item can also be heated during 3D printing. However, the receiver item may also be cooled during 3D printing.

The phrase "printing on a receiver item" and similar phrases include amongst others directly printing on the receiver item, or printing on a coating on the receiver item, or printing on 3D printed material earlier printed on the receiver item. The term "receiver item" may refer to a printing platform, a print bed, a substrate, a support, a build plate, or a building platform, etc... Instead of the term "receiver item" also the term "substrate" may be used. The phrase "printing on a receiver item" and similar phrases include amongst others also printing on a separate substrate on or comprised by a printing platform, a print bed, a support, a build plate, or a building platform, etc... Therefore, the phrase "printing on a substrate" and similar phrases include amongst others directly printing on the substrate, or printing on a coating on the substrate or printing on 3D printed material earlier printed on the substrate. Here below, further the term substrate is used, which may refer to a printing platform, a print bed, a substrate, a support, a build plate, or a building platform, etc., or a separate substrate thereon or comprised thereby.

Layer by layer printable material is deposited, by which the 3D printed item is generated (during the printing stage). The 3D printed item may show a characteristic ribbed structures (originating from the deposited filaments). However, it may also be possible that after a printing stage, a further stage is executed, such as a finalization stage. This stage may include removing the printed item from the receiver item and/or one or more post processing actions. One or more post processing actions may be executed before removing the printed item from the receiver item and/or one more post processing actions may be executed after removing the printed item from the receiver item. Post processing may include e.g. one or more of polishing, coating, adding a functional component, etc... Post-processing may include smoothening the ribbed structures, which may lead to an essentially smooth surface.

Further, the invention relates to a software product that can be used to execute the method described herein. Therefore, in yet a further aspect the invention also provides a computer program product, when running on a computer which is functionally coupled to or comprised by a fused deposition modeling 3D printer, is capable of bringing about the method as described herein.

Hence, in an aspect the invention (thus) provides a software product, which, when running on a computer is capable of bringing about (one or more embodiments of) the method (for producing a 3D item by means of fused deposition modelling) as described herein.

The herein described method provides 3D printed items. Hence, the invention also provides in a further aspect a 3D printed item obtainable with the herein described method.

In a further aspect a 3D printed item obtainable with the herein described method is provided. Especially, the invention provides an 3D item comprising 3D printed material. Especially, the 3D item may comprise a plurality of layers of 3D printed material, wherein at least part of the plurality of layers may comprise 3D printed material comprising (i) a polymeric matrix material, and (ii) a reflective material. As indicated above, especially the polymeric matrix material may be transmissive for UV radiation. Further, as indicated above, especially the reflective material is reflective for the UV radiation. Further, the reflective material may be at least partly enclosed by the polymeric matrix material. The reflective material may comprise a first fluoropolymer. Hence, in embodiments the invention provides a 3D item comprising 3D printed material, wherein the 3D item may comprise a plurality of layers of 3D printed material, wherein at least part of the plurality of layers may comprise 3D printed material comprising (i) a polymeric matrix material that is transmissive for UV radiation, and (ii) a reflective material that is reflective for the UV radiation and that is at least partly enclosed by the polymeric matrix material; wherein the reflective material may comprise a first fluoropolymer.

The 3D printed item may comprise a plurality of layers on top of each other, i.e. stacked layers. The width (thickness) and height of (individually 3D printed) layers may e.g. in embodiments be selected from the range of 100 - 5000 µm, such as 200-2500 µm, with the height in general being smaller than the width. For instance, the ratio of height and width may be equal to or smaller than 0.8, such as equal to or smaller than 0.6.

Layers may be core-shell layers or may consist of a single material. Within a layer, there may also be a change in composition, for instance when a core-shell printing process was applied and during the printing process it was changed from printing a first material (and not printing a second material) to printing a second material (and not printing the first material).

At least part of the 3D printed item may include a coating.

Some specific embodiments in relation to the 3D printed item have already been elucidated above when discussing the method. Below, some specific embodiments in relation to the 3D printed item are discussed in more detail.

The first fluoropolymer may comprise a microporous and or polycrystalline polytetrafluoroethylene. Further, in embodiments at least part of the plurality of layers may comprise core-shell 3D printed material, wherein the core-shell 3D printed material may comprise a core and a shell, wherein the core may comprise a fiber comprising the reflective material, and wherein the shell may comprise the polymeric matrix material.

The first fluoropolymer may comprise pores having one or more dimensions selected from the range of 1-40 µm. However, other porosities may also be possible.

Especially, the 3D printed material may comprise particulate material comprising the reflective material. The particulate material may be embedded in the matrix material.

The particulate material may (further) comprise a second reflective material selected from the group of BaSO₄ particles and reflective flakes. Alternatively or additionally, the second reflective material may be selected from the group of TiO₂ particles, Al₂O₃ particles, silver particles, and aluminum particles. Reflective flakes may e.g. comprise a silver coating or an aluminum coating.

Further, in embodiments the 3D printed material may comprise a core and a shell, wherein the core may comprise the reflective material and wherein the shell may comprise the matrix material.

Further, in specific embodiments one or more of the plurality of layers of the 3D printed material may have a width (w1) and a height (h1), which may individually be selected from the range of 0.1-10 mm, such as in the range of 0.1-5 mm, like in specific embodiments in the range of 0.1-3 mm.

The shell may have a largest shell width (wₛₘ₁). Especially, the largest shell width (wₛₘ₁) may be selected from the range of 2-15% of the width (w1).

Hence, in specific embodiments the 3D printed material may comprise particulate material comprising the reflective material, wherein the particulate material is embedded in the matrix material; wherein the particulate material may comprise a second reflective material selected from the group of BaSO₄ particles, TiO₂ particles, Al₂O₃ particles, silver particles, aluminum particles, and reflective flakes; wherein the 3D printed material comprising a core and a shell, wherein the core may comprise the reflective material and wherein the shell may comprise the matrix material; and wherein one or more of the plurality of layers of the 3D printed material have a width (w1) and a height (h1), individually selected from the range of 0.1-10 mm, like at maximum 8 mm, such as in embodiments 0.1-3 mm; wherein the shell has a largest shell width (wₛₘ₁), wherein the largest shell width (wₛₘ₁) is selected from the range of 2-15% of the width (w1).

The (with the herein described method) obtained 3D printed item may be functional *per se.* For instance, the 3D printed item may be a lens, a collimator, a reflector, etc... The thus obtained 3D item may (alternatively) be used for decorative or artistic purposes. The 3D printed item may include or be provided with a functional component. The functional component may especially be selected from the group consisting of an optical component, an electrical component, and a magnetic component. The term "optical component" especially refers to a component having an optical functionality, such as a lens, a mirror, a light transmissive element, an optical filter, etc... The term optical component may also refer to a light source (like a LED). The term "electrical component" may e.g. refer to an integrated circuit, PCB, a battery, a driver, but also a light source (as a light source may be considered an optical component and an electrical component), etc. The term magnetic component may e.g. refer to a magnetic connector, a coil, etc... Alternatively, or additionally, the functional component may comprise a thermal component (e.g. configured to cool or to heat an electrical component). Hence, the functional component may be configured to generate heat or to scavenge heat, etc...

As indicated above, the 3D printed item maybe used for different purposes. Amongst others, the 3D printed item maybe used in lighting. Hence, in yet a further aspect the invention also provides a lighting device comprising the 3D item as defined herein. In a specific aspect the invention provides a lighting system comprising (a) a light source configured to provide (visible) light source light and (b) the 3D item as defined herein, wherein 3D item may be configured as one or more of (i) at least part of a housing, (ii) at least part of a wall of a lighting chamber, and (iii) a functional component, wherein the functional component may be selected from the group consisting of an optical component, a support, an electrically insulating component, an electrically conductive component, a thermally insulating component, and a thermally conductive component. Hence, in specific embodiments the 3D item may be configured as one or more of (i) at least part of a lighting device housing, (ii) at least part of a wall of a lighting chamber, and (iii) an optical element. As a relative smooth surface may be provided, the 3D printed item may be used as mirror or lens, etc... The 3D item may be configured as shade. A device or system may comprise a plurality of different 3D printed items, having different functionalities.

The 3D item may comprise a hollow reflector.

Hence, in an aspect the invention also provides a lighting device comprising the 3D item as defined herein, wherein the 3D item is configured as one or more of (i) at least part of a lighting device housing, (ii) at least part of a wall of a lighting chamber, and (iii) an optical element, wherein the lighting device may comprise a light source configured to generate UV radiation having the one or more wavelengths selected from the range of 190-380 nm wherein the 3D item is configured downstream of the light source.

Returning to the 3D printing process, a specific 3D printer may be used to provide the 3D printed item described herein. Therefore, in yet a further aspect the invention also provides a fused deposition modeling 3D printer, comprising (a) a printer head comprising a printer nozzle, and (b) a 3D printable material providing device configured to provide 3D printable material to the printer head, wherein the fused deposition modeling 3D printer is configured to provide said 3D printable material by executing the herein described method.

The printer nozzle may include a single opening. In other embodiments, the printer nozzle may be of the core-shell type, having two (or more) openings. The term "printer head" may also refer to a plurality of (different) printer heads; hence, the term "printer nozzle" may also refer to a plurality of (different) printer nozzles.

The 3D printable material providing device may provide a filament comprising 3D printable material to the printer head or may provide the 3D printable material as such, with the printer head creating the filament comprising 3D printable material. Hence, in embodiments the invention provides a fused deposition modeling 3D printer, comprising (a) a printer head comprising a printer nozzle, and (b) a filament providing device configured to provide a filament comprising 3D printable material to the printer head, wherein the fused deposition modeling 3D printer is configured to provide said 3D printable material to a substrate, and wherein the 3D printer is configured to execute in an operational mode the herein described method.

Especially, the 3D printer may comprise a controller (or is functionally coupled to a controller) that is configured to execute in a controlling mode (or "operation mode") the method as described herein. Instead of the term "controller" also the term "control system" (see e.g. above) may be applied.

The term "controlling" and similar terms especially refer at least to determining the behavior or supervising the running of an element. Hence, herein "controlling" and similar terms may e.g. refer to imposing behavior to the element (determining the behavior or supervising the running of an element), etc., such as e.g. measuring, displaying, actuating, opening, shifting, changing temperature, etc.. Beyond that, the term "controlling" and similar terms may additionally include monitoring. Hence, the term "controlling" and similar terms may include imposing behavior on an element and also imposing behavior on an element and monitoring the element. The controlling of the element can be done with a control system, which may also be indicated as "controller". The control system and the element may thus at least temporarily, or permanently, functionally be coupled. The element may comprise the control system. The control system and element may not be physically coupled. Control can be done via wired and/or wireless control. The term "control system" may also refer to a plurality of different control systems, which especially are functionally coupled, and of which e.g. one control system may be a master control system and one or more others may be slave control systems. A control system may comprise or may be functionally coupled to a user interface.

The control system may also be configured to receive and execute instructions form a remote control. The control system may be controlled via an App on a device, such as a portable device, like a Smartphone or I-phone, a tablet, etc.. The device is thus not necessarily coupled to the lighting system, but may be (temporarily) functionally coupled to the lighting system.

Hence, in embodiments the control system may (also) be configured to be controlled by an App on a remote device. In such embodiments the control system of the lighting system may be a slave control system or control in a slave mode. For instance, the lighting system may be identifiable with a code, especially a unique code for the respective lighting system. The control system of the lighting system may be configured to be controlled by an external control system which has access to the lighting system on the basis of knowledge (input by a user interface of with an optical sensor (e.g. QR code reader) of the (unique) code. The lighting system may also comprise means for communicating with other systems or devices, such as on the basis of Bluetooth, WIFI, LiFi, ZigBee, BLE or WiMAX, or another wireless technology.

The system, or apparatus, or device may execute an action in a "mode" or "operation mode" or "mode of operation". Likewise, in a method an action or stage, or step may be executed in a "mode" or "operation mode" or "mode of operation" or "operational mode". The term "mode" may also be indicated as "controlling mode". This does not exclude that the system, or apparatus, or device may also be adapted for providing another controlling mode, or a plurality of other controlling modes. Likewise, this may not exclude that before executing the mode and/or after executing the mode one or more other modes may be executed.

However, in embodiments a control system may be available, that is adapted to provide at least the controlling mode. Would other modes be available, the choice of such modes may especially be executed via a user interface, though other options, like executing a mode in dependence of a sensor signal or a (time) scheme, may also be possible. The operation mode may in embodiments also refer to a system, or apparatus, or device, that can only operate in a single operation mode (i.e. "on", without further tunability).

Hence, the control system may control in dependence of one or more of an input signal of a user interface, a sensor signal (of a sensor), and a timer. The term "timer" may refer to a clock and/or a predetermined time scheme.

Instead of the term "fused deposition modeling (FDM) 3D printer" shortly the terms "3D printer", "FDM printer" or "printer" may be used. The printer nozzle may also be indicated as "nozzle" or sometimes as "extruder nozzle".

In yet a further aspect, the invention also provides a method for treating air or a surface (e.g. external from the 3D item as described herein), wherein the method may comprise exposing air to UV radiation from the lighting device (as described herein).

### BRIEF DESCRIPTION OF THE DRAWINGS

Embodiments of the invention will now be described, by way of example only, with reference to the accompanying schematic drawings in which corresponding reference symbols indicate corresponding parts, and in which:
Figs. 1a-1c schematically depict some general aspects of the 3D printer and of an embodiment of 3D printed material;
Fig. 2a-2b schematically depict some further aspects;
Fig. 3 schematically depicts an application;
Fig. 4 schematically depicts a further embodiment;
Fig. 5 show some possible fluoropolymers. The schematic drawings are not necessarily to scale.

### DETAILED DESCRIPTION OF THE EMBODIMENTS

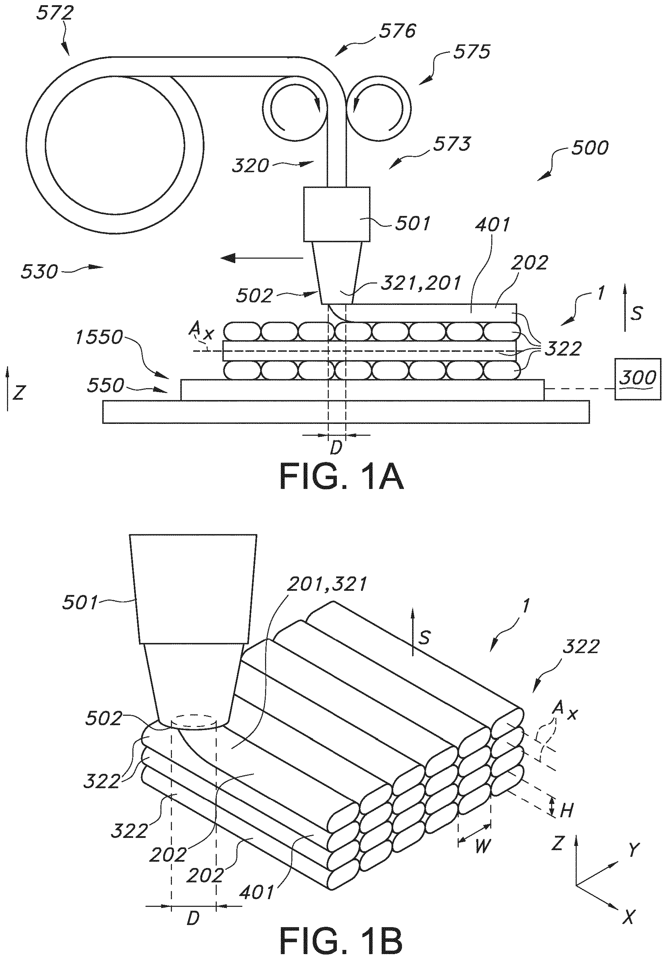

Fig. 1a schematically depicts some aspects of the 3D printer. Reference 500 indicates a 3D printer. Reference 530 indicates the functional unit configured to 3D print, especially FDM 3D printing; this reference may also indicate the 3D printing stage unit. Here, only the printer head for providing 3D printed material, such as an FDM 3D printer head is schematically depicted. Reference 501 indicates the printer head. The 3D printer of the present invention may especially include a plurality of printer heads (see below). Reference 502 indicates a printer nozzle. The 3D printer of the present invention may especially include a plurality of printer nozzles, though other embodiments are also possible. Reference 320 indicates a filament of printable 3D printable material (such as indicated above).

Instead of a filament also pellets may be used as 3D printable material. Both can be extruded via the printer nozzle.

For the sake of clarity, not all features of the 3D printer have been depicted, only those that are of especial relevance for the present invention (see further also below). Reference 321 indicates extrudate (of 3D printable material 201).

The 3D printer 500 is configured to generate a 3D item 1 by layer-wise depositing on a receiver item 550, which may in embodiments at least temporarily be cooled, a plurality of layers 322 wherein each layers 322 may comprise 3D printable material 201, such as having a melting temperature Tₘ. The 3D printable material 201 may be deposited on a substrate 1550 (during the printing stage). By deposition, the 3D printable material 201 has become 3D printed material 202. 3D printable material 201 escaping from the nozzle 502 is also indicated as extrudate 321. Reference 401 indicates thermoplastic material.

The 3D printer 500 may be configured to heat the filament 320 material upstream of the printer nozzle 502. This may e.g. be done with a device comprising one or more of an extrusion and/or heating function. Such device is indicated with reference 573, and is arranged upstream from the printer nozzle 502 (i.e. in time before the filament material leaves the printer nozzle 502). The printer head 501 may (thus) include a liquefier or heater. Reference 201 indicates printable material. When deposited, this material is indicated as (3D) printed material, which is indicated with reference 202.

Reference 572 indicates a spool or roller with material, especially in the form of a wire, which may be indicated as filament 320. The 3D printer 500 transforms this in an extrudate 321 downstream of the printer nozzle which becomes a layer 322 on the receiver item or on already deposited printed material. In general, the diameter of the extrudate 321 downstream of the nozzle 502 is reduced relative to the diameter of the filament 322 upstream of the printer head 501. Hence, the printer nozzle is sometimes (also) indicated as extruder nozzle. Arranging layer 322 by layer 322 and/or layer 322t on layer 322, a 3D item 1 may be formed. Reference 575 indicates the filament providing device, which here amongst others include the spool or roller and the driver wheels, indicated with reference 576.

Reference Ax indicates a longitudinal axis or filament axis.

Reference 300 schematically depicts a control system. The control system may be configured to control the 3D printer 500. The control system 300 may be comprised or functionally coupled to the 3D printer 500. The control system 300 may further comprise or be functionally coupled to a temperature control system configured to control the temperature of the receiver item 550 and/or of the printer head 501. Such temperature control system may include a heater which is able to heat the receiver item 550 to at least a temperature of 50 °C, but especially up to a range of about 350 °C, such as at least 200 °C.

Alternatively or additionally, in embodiments the receiver plate may also be moveable in one or two directions in the x-y plane (horizontal plane). Further, alternatively or additionally, in embodiments the receiver plate may also be rotatable about z axis (vertical). Hence, the control system may move the receiver plate in one or more of the x-direction, y-direction, and z-direction.

Alternatively, the printer can have a head can also rotate during printing. Such a printer has an advantage that the printed material cannot rotate during printing.

Layers are indicated with reference 322, and have a layer height H and a layer width W.

Note that the 3D printable material is not necessarily provided as filament 320 to the printer head. Further, the filament 320 may also be produced in the 3D printer 500 from pieces of 3D printable material.

Reference D indicates the diameter of the nozzle (through which the 3D printable material 201 is forced).

Fig. 1b schematically depicts in 3D in more detail the printing of the 3D item 1 under construction. Here, in this schematic drawing the ends of the filaments 321 in a single plane are not interconnected, though in reality this may in embodiments be the case. Reference H indicates the height of a layer. Layers are indicated with reference 203. Here, the layers have an essentially circular cross-section. Often, however, they may be flattened, such as having an outer shape resembling a flat oval tube or flat oval duct (i.e. a circular shaped bar having a diameter that is compressed to have a smaller height than width, wherein the sides (defining the width) are (still) rounded).

Hence, Figs. 1a-1b schematically depict some aspects of a fused deposition modeling 3D printer 500, comprising (a) a first printer head 501 comprising a printer nozzle 502, (b) a filament providing device 575 configured to provide a filament 321 comprising 3D printable material 201 to the first printer head 501, and optionally (c) a receiver item 550. In Figs. 1a-1b, the first or second printable material or the first or second printed material are indicated with the general indications printable material 201 and printed material 202, respectively. Directly downstream of the nozzle 502, the filament 321 with 3D printable material becomes, when deposited, layer 322 with 3D printed material 202.

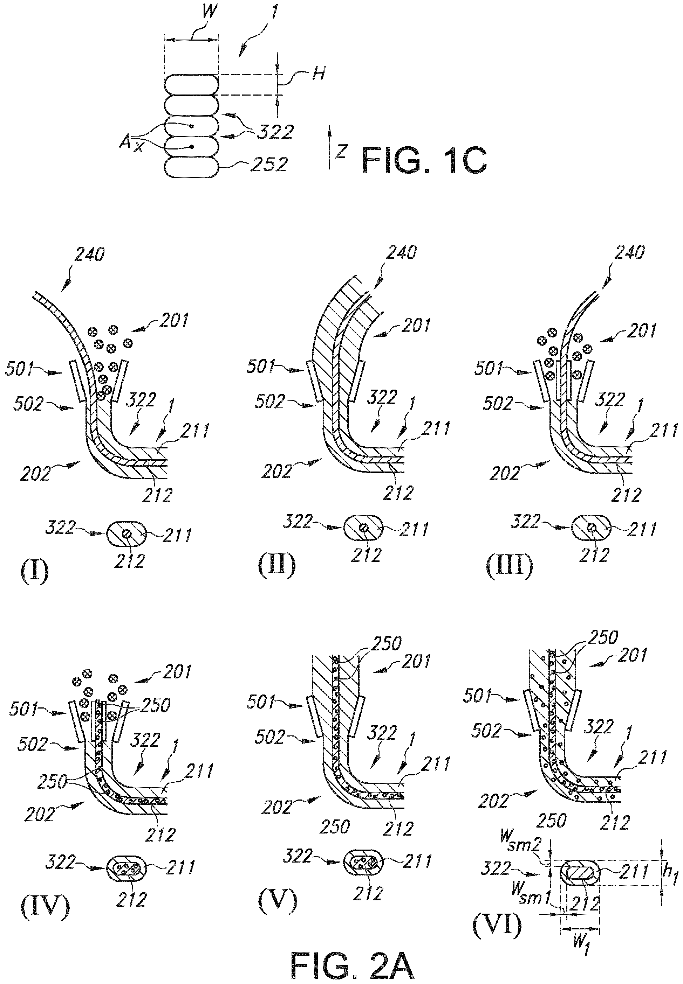

Fig. 1c schematically depicts a stack of 3D printed layers 322, each having a layer height H and a layer width W. Note that in embodiments the layer width and/or layer height may differ for two or more layers 322. Reference 252 in Fig. 1c indicates the item surface of the 3D item (schematically depicted in Fig. 1c).

Referring to Figs. 1a-1c, the filament of 3D printable material that is deposited leads to a layer having a height H (and width W). Depositing layer 322 after layer 322, the 3D item 1 is generated. Fig. 1c very schematically depicts a single-walled 3D item 1.

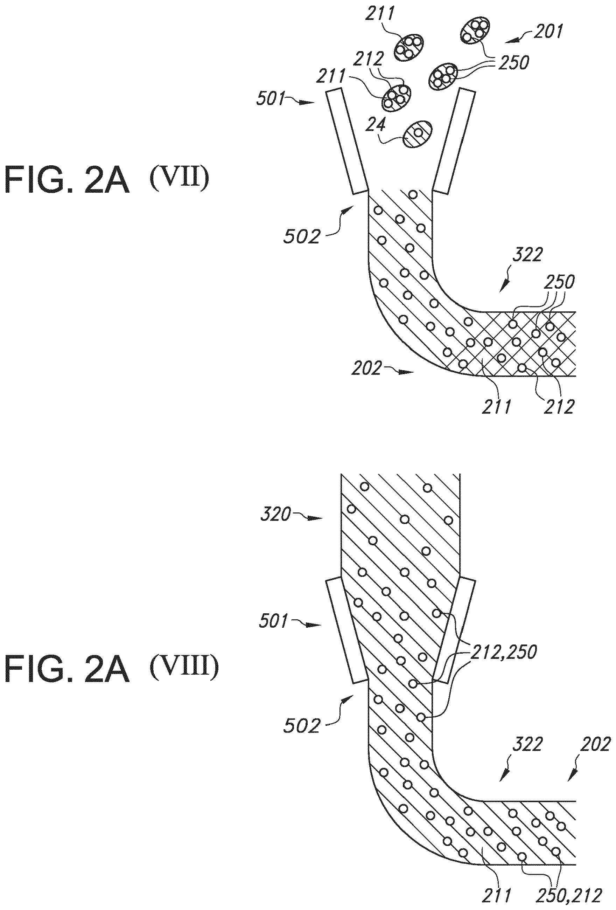

Fig. 2a very schematically depicts a number of embodiments how 3D printable material can be printed.

Amongst others, the invention provides a method for producing a 3D item 1 by means of fused deposition modelling. The method may comprise a 3D printing stage, wherein the 3D printing stage may comprise a reflective material deposition stage. Especially, the reflective material deposition stage may comprise: (a) providing 3D printable material 201 comprising (i) a polymeric matrix material 211 that is transmissive for UV radiation, wherein the polymeric matrix material 211 may comprise thermoplastic material, and (ii) a reflective material 212 that is reflective for the UV radiation and that is at least partly enclosed by the polymeric matrix material 211. Especially, the reflective material 212 may comprise a first fluoropolymer. Further, the reflective material deposition stage may comprise: (b) depositing the 3D printable material 201, to provide the 3D item 1 comprising 3D printed material 202 comprising the matrix material 211 and the reflective material 212.

The first fluoropolymer may comprise a (first) microporous fluoropolymer. The first fluoropolymer may comprise a microporous polytetrafluoroethylene. The first fluoropolymer may comprise microporous first fluoropolymer comprising pores having one or more dimensions selected from the range of 1-40 µm. For instance, the medial pore size may be 1-10 µm.

The first fluoropolymer may comprise a first melting temperature T_{f1,m}, wherein the matrix material 211 may comprise a second fluoropolymer having a second fluoropolymer melting temperature T_{f2,m}, or in the case of amorphous polymer T_{f2,g} wherein T_{f2,m}≤T_{f1,m}-10°C or T_{f2,g}≤T_{f1,m}-10°C .

Referring to embodiments I-III in Fig. 2a, the reflective material deposition stage may comprise a guiding a fiber 240 (without melting) through a 3D printer nozzle 502, while also b providing the polymeric matrix material 211 to the 3D printer nozzle 502 to provide core-shell 3D printed material 202.

Referring to embodiments IV-VI, the 3D printable material 201 may comprise particulate material 250 comprising the reflective material 212, wherein the particulate material 250 is embedded in the matrix material 211.

The particulate material 250 may comprise a second reflective material 250 selected from the group of BaSO₄ particles, TiO₂ particles, Al₂O₃ particles, silver particles, aluminum particles, and reflective flakes.

As schematically depicted, the method may comprise depositing 3D printable material 201 comprising a core 260 and a shell 270, wherein the core 260 may comprise the reflective material 212 (wherein the core 260 may comprise thermoplastic material,) and wherein the shell 270 may comprise the matrix material 211; wherein a layer 322 of the 3D printed material 202 has a width w1 and a height h1, individually selected from the range of 0.1-10 mm, like at maximum 8 mm, such as in embodiments 0.1-3 mm; wherein the shell 270 has a largest shell width wₛₘ₁, wherein the largest shell width wₛₘ₁ is selected from the range of 2-15% of the width w1. There may also be a minimum width, which is indicated with reference wₛₘ₂. The minimum with may be zero, but may also be non-zero, like at least 10% of wₛₘ₁. Referring to embodiment VI of Fig. 2a, the cross-section parameters may be defined along coinciding lines.

Embodiments VII and VIII schematically depict some further embodiments. In embodiment VII the 3D printable material 201 is provided as printable material pellets, which may be deformed in the printer head, especially the printer nozzle 502. The pellets of 3D printable material 201 may comprise the matrix material 211 that may comprise thermoplastic material, more especially may consist of thermoplastic material, with reflective material 212 in the form of particles embedded therein. In this way, a layer 322 of 3D printed material 202 is provided, wherein the particulate reflective material 212 may be available. In embodiment VIII a filament 320 is provided, which may comprise the matrix material 211 that may comprise thermoplastic material, more especially may consist of thermoplastic material, with reflective material 212 in the form of particles embedded therein.

The polymeric matrix material 211 may be transmissive for UV radiation having one or more wavelengths selected from the range of 190-380 nm, and the reflective material 212 may be reflective for the UV radiation having the one or more wavelengths selected from the range of 190-380 nm; wherein the polymeric matrix material, the reflective material, the relative amounts, the thickness (i.e. the width w1) of the 3D printed layer are selected such that under perpendicular radiation with the UV radiation having the one or more wavelengths selected from the range of 190-380 nm a layer 322 comprising the 3D printed material 202 reflects at least 40% of the UV radiation having the one or more wavelengths selected from the range of 190-380 nm.

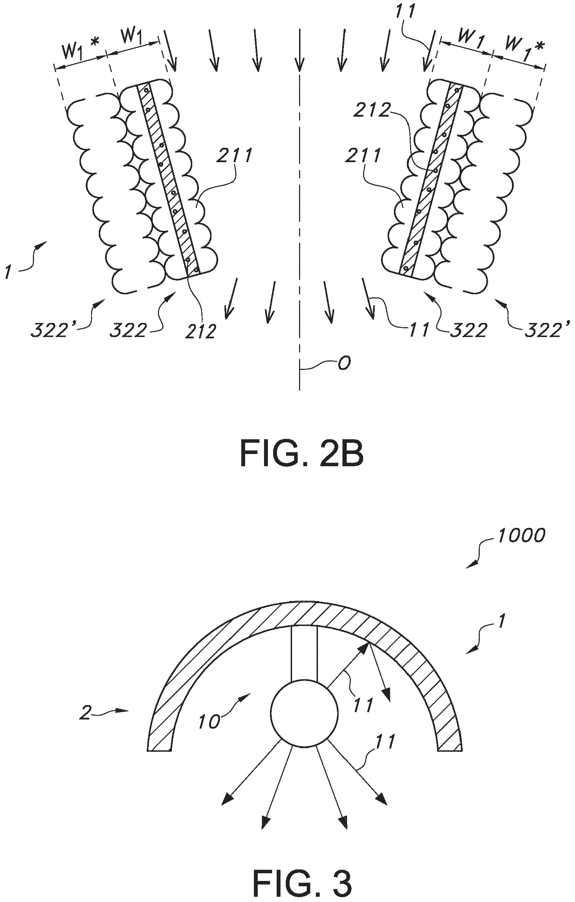

Referring to Fig. 2b, the 3D item 1 may comprise a hollow reflector, such as a reflective collimator. Reference 11 indicates radiation, such as UV radiation. Reference O indicates an optical axis. The 3D item 1 may e.g. collimate the radiation 11. Fig. 2b also schematically depicts an embodiment wherein part of the 3D printed item is printed according to the reflective material deposition stage. The result thereof is (are) layer(s) 322. These may provide the inner surface of the 3D items 1 reflectivity for UV radiation 11. Another part of the 3D item may comprise a different type of 3D printed material 202. This layer / these layer(s) are indicated with reference 322'. The width w1 (and/or the heights) may be different, but may also be the same. The width of the layer 322 printed according to the reflective material deposition stage is indicated with reference w1 and the width of the layer 322' not printed according to the reflective material deposition stage is indicated with reference w1'. For instance, the layer 322' not printed according to the reflective material deposition stage may be (visible) light absorbing and/or may have other optical properties than the layers 322 printed according to the reflective material deposition stage.

Referring to e.g. Fig. 2b, the invention also provides a 3D item 1 comprising 3D printed material 202, wherein the 3D item 1 may comprise a plurality of layers 322 of 3D printed material 202, wherein at least part of the plurality of layers 322 may comprise 3D printed material 202 comprising (i) a polymeric matrix material 211 that is transmissive for UV radiation, and (ii) a reflective material 212 that is reflective for the UV radiation and that is at least partly enclosed by the polymeric matrix material 211; wherein the reflective material 212 may comprise a first fluoropolymer.

The first fluoropolymer may comprise a microporous polytetrafluoroethylene, and wherein the first fluoropolymer may comprise pores having one or more dimensions selected from the range of 1-40 µm.

In embodiments, at least part of the plurality of layers 322 may comprise core-shell 3D printed material 202, wherein the core-shell 3D printed material 202 may comprise a core 260 and a shell 270, wherein the core 260 may comprise a fiber 240 comprising the reflective material 212, and wherein the shell 270 may comprise the polymeric matrix material 211.

The 3D printed material 202 may comprise particulate material 250 comprising the reflective material 212, wherein the particulate material 250 is embedded in the matrix material 211; wherein the particulate material 250 may comprise a second reflective material 250 selected from the group of BaSO₄ particles, TiO₂ particles, Al₂O₃ particles, silver particles, aluminum particles, and reflective flakes; wherein the 3D printed material 201 comprising a core 260 and a shell 270, wherein the core 260 may comprise the reflective material 212 and wherein the shell 270 may comprise the matrix material 211; and wherein one or more of the plurality of layers 322 of the 3D printed material 202 have a width w1 and a height h1, individually selected from the range of 0.1-10 mm, like at maximum 8 mm, such as in embodiments 0.1-3 mm; wherein the shell 270 has a largest shell width wₛₘ₁, wherein the largest shell width wₛₘ₁ is selected from the range of 2-15% of the width w1.

Fig. 3 schematically depicts an embodiment of a lamp or luminaire, indicated with reference 2, which may comprise a light source 10 for generating light 11. The lamp may comprise a housing or shade or another element, which may comprise or be the 3D printed item 1. Here, the half sphere (in cross-sectional view) schematically indicates a housing or shade. The lamp or luminaire may be or may comprise a lighting device 1000 (which may comprise the light source 10). Hence, in specific embodiments the lighting device 1000 may comprise the 3D item 1. The 3D item 1 may be configured as one or more of (i) at least part of a lighting device housing, (ii) at least part of a wall of a lighting chamber, and (iii) an optical element. Hence, the 3D item may in embodiments be reflective for light source light 11 and/or transmissive for light source light 11. Here, the 3D item may e.g. be a housing or shade.

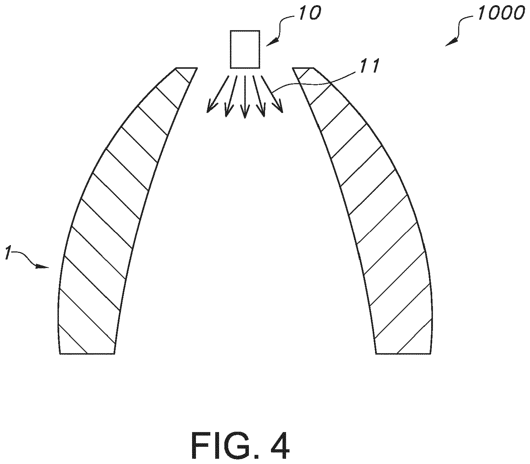

However, the 3D item 1 may also be a (hollow) reflector, like a collimator. An example is shown in Fig. 4.

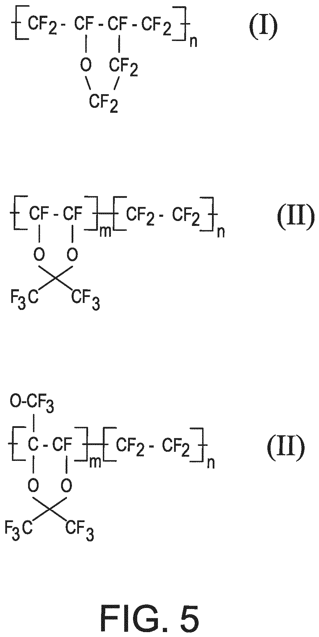

Fig. 5 show some possible chemical formulas of various amorphous fluoropolymers. Referring to formula I, Cytop may have a glass transition temperature T_{g} of about 108°C. Referring to formula II, Teflon AF (1600) may have a T_{g} of about 160 °C and Teflon AF (2400) may have a T_{g} of about 240 °C. Referring to formula III, Hyflon AD 40H may have a glass transition temperature T_{g} of about 90°C. Hence, for instance Poly(tetrafluoroethylene-co-2,2,4-trifluoro-5-trifluoromethoxy-1,3-dioxole), amorphous perfluoropolymer, P(TTD-TFE), TTD-TFE copolymers may be applied, but other type of materials may also be applied. Further, examples of fluoropolymers are e.g. PTFE, PCTFE, FEP, PVF, PVDF, ECTFE, PFA, ETFE, THV. Especially ETFE may have (also) very good optical results.

The term "plurality" refers to two or more. The terms "substantially" or "essentially" herein, and similar terms, will be understood by the person skilled in the art. The terms "substantially" or "essentially" may also include embodiments with "entirely", "completely", "all", etc. Hence, in embodiments the adjective substantially or essentially may also be removed. Where applicable, the term "substantially" or the term "essentially" may also relate to 90% or higher, such as 95% or higher, especially 99% or higher, even more especially 99.5% or higher, including 100%. The term "comprise" also includes embodiments wherein the term "comprises" means "consists of". The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species". Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices, apparatus, or systems may herein amongst others be described during operation. As will be clear to the person skilled in the art, the invention is not limited to methods of operation, or devices, apparatus, or systems in operation.

It should be noted that the above-mentioned embodiments illustrate rather than limit the invention, and that those skilled in the art will be able to design many alternative embodiments without departing from the scope of the appended claims.

**In** the claims, any reference signs placed between parentheses shall not be construed as limiting the claim.

Use of the verb "to comprise" and its conjugations does not exclude the presence of elements or steps other than those stated in a claim. Unless the context clearly requires otherwise, throughout the description and the claims, the words "comprise", "comprising", and the like are to be construed in an inclusive sense as opposed to an exclusive or exhaustive sense; that is to say, in the sense of "including, but not limited to". The article "a" or "an" preceding an element does not exclude the presence of a plurality of such elements.

The invention may be implemented by means of hardware comprising several distinct elements, and by means of a suitably programmed computer. In a device claim, or an apparatus claim, or a system claim, enumerating several means, several of these means may be embodied by one and the same item of hardware. The mere fact that certain measures are recited in mutually different dependent claims does not indicate that a combination of these measures cannot be used to advantage. The invention also provides a control system that may control the device, apparatus, or system, or that may execute the herein described method or process. Yet further, the invention also provides a computer program product, when running on a computer which is functionally coupled to or comprised by the device, apparatus, or system, controls one or more controllable elements of such device, apparatus, or system. The invention further applies to a device, apparatus, or system comprising one or more of the characterizing features described in the description and/or shown in the attached drawings. The invention further pertains to a method or process comprising one or more of the characterizing features described in the description and/or shown in the attached drawings.

The various aspects discussed in this patent can be combined in order to provide additional advantages. Further, the person skilled in the art will understand that embodiments can be combined, and that also more than two embodiments can be combined. Furthermore, some of the features can form the basis for one or more divisional applications.

It goes without saying that one or more of the first (printable or printed) material and second (printable or printed) material may contain fillers such as glass and fibers which do not have (to have) influence on the on T_{g} or Tₘ of the material(s).

## Claims

1. A method for producing a 3D item (1) by means of fused deposition modelling, the method comprising a 3D printing stage, wherein the 3D printing stage comprises a reflective material deposition stage, wherein the reflective material deposition stage comprises:
- providing 3D printable material (201) comprising (i) a polymeric matrix material (211) that is transmissive for UV radiation, wherein the polymeric matrix material (211) comprises thermoplastic material, and (ii) a reflective material (212) that is reflective for the UV radiation and that is at least partly enclosed by the polymeric matrix material (211); wherein the reflective material (212) comprises a first fluoropolymer; and
- depositing the 3D printable material (201), to provide the 3D item (1) comprising 3D printed material (202) comprising the matrix material (211) and the reflective material (212);
**characterized in that** the first fluoropolymer comprises a first microporous polytetrafluoroethylene.

2. The method according to claim 1, wherein the first fluoropolymer comprises a microporous fluoropolymer, having a porosity selected from the range about 35-45%,

3. The method according to any one of the preceding claims, wherein the first fluoropolymer comprises a first amorphous fluoropolymer.

4. The method according to any one of the preceding claims, wherein the first fluoropolymer comprises a first fluoropolymer melting temperature T_{f1,m}, wherein the matrix material (211) comprises a second fluoropolymer having a second fluoropolymer melting temperature T_{f2,m} and/or a second fluoropolymer glass transition temperature T_{f2,g}, wherein T_{f2,m}≤T_{f1,m}-10°C and/or wherein T_{f2,g}≤T_{f1,m}-10°C.

5. The method according to any one of the preceding claims 1-4, wherein the reflective material deposition stage comprises (a) guiding a fiber (240) without melting through a 3D printer nozzle (502), while also (b) providing the polymeric matrix material (211) to the 3D printer nozzle (502) to provide core-shell 3D printed material (202).

6. The method according to any one of the preceding claims 1-4, wherein the 3D printable material (201) comprises particulate material (250) comprising the reflective material (212), wherein the particulate material (250) is embedded in the matrix material (211).

7. The method according to claim 6, wherein the particulate material (250) comprises a second reflective material (250) selected from the group of BaSO₄ particles, TiO₂ particles, Al₂O₃ particles, silver particles, aluminum particles, and reflective flakes.

8. The method according to any one of the preceding claims 5-7, comprising depositing 3D printable material (201) comprising a core (260) and a shell (270), wherein the core (260) comprises the reflective material (212), wherein the core (260) comprises thermoplastic material, and wherein the shell (270) comprises the matrix material (211); wherein a layer (322) of the 3D printed material (202) has a width (w1) and a height (h1), individually selected from the range of 0.1-10 mm; wherein the shell (270) has a largest shell width (wₛₘ₁), wherein the largest shell width (wₛₘ₁) is selected from the range of 2-15% of the width (w1).

9. The method according to any one of the preceding claims, wherein the 3D item (1) comprises a hollow reflector.

10. A 3D item (1) comprising 3D printed material (202), wherein the 3D item (1) comprises a plurality of layers (322) of 3D printed material (202), wherein at least part of the plurality of layers (322) comprises 3D printed material (202) comprising (i) a polymeric matrix material (211) that is transmissive for UV radiation, and (ii) a reflective material (212) that is reflective for the UV radiation and that is at least partly enclosed by the polymeric matrix material (211); wherein the reflective material (212) comprises a first fluoropolymer, **characterized in that** the first fluoropolymer comprises a first microporous polytetrafluoroethylene.

11. The 3D item (1) according to claim 10, wherein at least part of the plurality of layers (322) comprises core-shell 3D printed material (202), wherein the core-shell 3D printed material (202) comprises a core (260) and a shell (270), wherein the core (260) comprises a fiber (240) comprising the reflective material (212), and wherein the shell (270) comprises the polymeric matrix material (211).

12. The 3D item (1) according to any one of the preceding claims 10-11, wherein the 3D printed material (202) comprises particulate material (250) comprising the reflective material (212), wherein the particulate material (250) is embedded in the matrix material (211); wherein the particulate material (250) comprises a second reflective material (250) selected from the group of BaSO₄ particles, TiO₂ particles, Al₂O₃ particles, silver particles, aluminum particles, and reflective flakes; wherein the 3D printed material (201) comprising a core (260) and a shell (270), wherein the core (260) comprises the reflective material (212) and wherein the shell (270) comprises the matrix material (211); and wherein one or more of the plurality of layers (322) of the 3D printed material (202) have a width (w1) and a height (h1), individually selected from the range of 0.1-10 mm; wherein the shell (270) has a largest shell width (wₛₘ₁), wherein the largest shell width (wₛₘ₁) is selected from the range of 2-15% of the width (w1).

13. A lighting device (1000) comprising the 3D item (1) according to any one of the preceding claims 10-12, wherein the 3D item (1) is configured as one or more of (i) at least part of a lighting device housing, (ii) at least part of a wall of a lighting chamber, and (iii) an optical element, wherein the lighting device (1000) comprises a light source (10) configured to generate UV radiation (11) having the one or more wavelengths selected from the range of 190-380 nm wherein the 3D item (1) is configured downstream of the light source (10).

## Patentansprüche

1. Verfahren zum Herstellen eines 3D-Objekts (1) mittels Schmelzschichtung, wobei das Verfahren einen 3D-Druckschritt umfasst, wobei der 3D-Druckschritt einen Schritt zum Abscheiden von reflektierendem Material umfasst, wobei der Schritt zum Abscheiden von reflektierendem Material umfasst:
- Bereitstellen eines 3D-druckbaren Materials (201), umfassend (i) ein Polymermatrixmaterial (211), das für UV-Strahlung durchlässig ist, wobei das Polymermatrixmaterial (211) thermoplastisches Material umfasst, und (ii) ein reflektierendes Material (212), das für die UV-Strahlung reflektierend ist und das mindestens teilweise von dem Polymermatrixmaterial (211) umschlossen ist; wobei das reflektierende Material (212) ein erstes Fluorpolymer umfasst; und
- Abscheiden des 3D-druckbaren Materials (201), um das 3D-Objekt (1) bereitzustellen, umfassend 3D-gedrucktes Material (202), umfassend das Matrixmaterial (211) und das reflektierende Material (212);
**dadurch gekennzeichnet, dass** das erste Fluorpolymer ein erstes mikroporöses Polytetrafluorethylen umfasst.

2. Verfahren nach Anspruch 1, wobei das erste Fluorpolymer ein mikroporöses Fluorpolymer umfasst, das eine Porosität, die aus dem Bereich von etwa 35-45 % ausgewählt ist, aufweist.

3. Verfahren nach einem der vorstehenden Ansprüche, wobei das erste Fluorpolymer ein erstes amorphes Fluorpolymer umfasst.

4. Verfahren nach einem der vorstehenden Ansprüche, wobei das erste Fluorpolymer eine erste Fluorpolymerschmelztemperatur T_{f1,m} umfasst, wobei das Matrixmaterial (211) ein zweites Fluorpolymer umfasst, das eine zweite Fluorpolymerschmelztemperatur T_{f2,m} und/oder eine zweite Fluorpolymerglasübergangstemperatur T_{f2,g} aufweist, wobei T_{f2,m}≤T_{f1,m}-10 °C und/oder wobei T_{f2,g}≤T_{f1,m}-10 °C.

5. Verfahren nach einem der vorstehenden Ansprüche 1 bis 4, wobei der Schritt des Abscheidens des reflektierenden Materials umfasst: (a) Führen einer Faser (240) ohne Schmelzen durch eine 3D-Druckerdüse (502), während ebenso (b) das polymere Matrixmaterial (211) der 3D-Druckerdüse (502) bereitgestellt wird, um 3D-gedrucktes Kern-Schale-Material (202) bereitzustellen.

6. Verfahren nach einem der vorstehenden Ansprüche 1 bis 4, wobei das 3D-druckbare Material (201) Partikelmaterial (250) umfasst, umfassend das reflektierende Material (212), wobei das Partikelmaterial (250) in das Matrixmaterial (211) eingebettet ist.

7. Verfahren nach Anspruch 6, wobei das Partikelmaterial (250) ein zweites reflektierendes Material (250) umfasst, das aus der Gruppe von BaSO₄-Partikeln, TiO₂-Partikeln, Al₂O₃-Partikeln, Silberpartikeln, Aluminiumpartikeln und reflektierende Flocken ausgewählt ist.

8. Verfahren nach einem der vorstehenden Ansprüche 5 bis 7, umfassend das Abscheiden von 3D-druckbarem Material (201), umfassend einen Kern (260) und eine Schale (270), wobei der Kern (260) das reflektierende Material (212) umfasst, wobei der Kern (260) thermoplastisches Material umfasst und wobei die Schale (270) das Matrixmaterial (211) umfasst; wobei eine Schicht (322) des 3D-gedruckten Materials (202) eine Breite (w1) und eine Höhe (h1) aufweist, die jeweils aus dem Bereich von 0,1-10 mm ausgewählt sind; wobei die Schale (270) eine größte Schalenbreite (wₛₘ₁) aufweist, wobei die größte Schalenbreite (wₛₘ₁) aus dem Bereich von 2-15 % der Breite (w1) ausgewählt ist.

9. Verfahren nach einem der vorstehenden Ansprüche, wobei das 3D-Objekt (1) einen hohlen Reflektor umfasst.

10. 3D-Objekt (1), umfassend 3D-gedrucktes Material (202), wobei das 3D-Objekt (1) eine Vielzahl von Schichten (322) aus 3D-gedrucktem Material (202) umfasst, wobei mindestens ein Teil der Vielzahl von Schichten (322) 3D-gedrucktes Material (202) umfasst, umfassend (i) ein polymeres Matrixmaterial (211), das für UV-Strahlung durchlässig ist, und (ii) ein reflektierendes Material (212), das für die UV-Strahlung reflektierend ist und das mindestens teilweise von dem polymeren Matrixmaterial (211) umschlossen ist; wobei das reflektierende Material (212) ein erstes Fluorpolymer umfasst, **dadurch gekennzeichnet, dass** das erste Fluorpolymer ein erstes mikroporöses Polytetrafluorethylen umfasst.

11. 3D-Objekt (1) nach Anspruch 10, wobei mindestens ein Teil der Vielzahl von Schichten (322) 3D-gedrucktes Kern-Schale-Material (202) umfasst, wobei das 3D-gedruckte Kern-Schale-Material (202) einen Kern (260) und eine Schale (270) umfasst, wobei der Kern (260) eine Faser (240) umfasst, umfassend das reflektierende Material (212), und wobei die Schale (270) das polymere Matrixmaterial (211) umfasst.

12. 3D-Objekt (1) nach einem der vorstehenden Ansprüche 10 bis 11, wobei das 3D-gedruckte Material (202) Partikelmaterial (250) umfasst, umfassend das reflektierende Material (212), wobei das Partikelmaterial (250) in das Matrixmaterial (211) eingebettet ist; wobei das Partikelmaterial (250) ein zweites reflektierendes Material (250) umfasst, das aus der Gruppe von BaSO₄-Partikeln, TiO₂-Partikeln, Al₂O₃-Partikeln, Silberpartikeln, Aluminiumpartikeln und reflektierenden Flocken ausgewählt ist; wobei das 3D-gedruckte Material (201) einen Kern (260) und eine Schale (270) umfasst, wobei der Kern (260) das reflektierende Material (212) umfasst und wobei die Schale (270) das Matrixmaterial (211) umfasst; und wobei eine oder mehrere der Vielzahl von Schichten (322) des 3D-gedruckten Materials (202) eine Breite (w1) und eine Höhe (h1) aufweisen, die einzeln aus dem Bereich von 0,1-10 mm ausgewählt sind; wobei die Schale (270) eine größte Schalenbreite (wₛₘ₁) aufweist, wobei die größte Schalenbreite (wₛₘ₁) aus dem Bereich von 2-15 % der Breite (w1) ausgewählt ist.

13. Beleuchtungsvorrichtung (1000), umfassend das 3D-Objekt (1) nach einem der vorstehenden Ansprüche 10 bis 12, wobei das 3D-Objekt (1) als eines oder mehrere konfiguriert ist von (i) mindestens einem Teil eines Beleuchtungsvorrichtungsgehäuses, (ii) mindestens einem Teil einer Wand einer Beleuchtungskammer und (iii) einem optischen Element, wobei die Beleuchtungsvorrichtung (1000) eine Lichtquelle (10) umfasst, die konfiguriert ist, um UV-Strahlung (11) zu erzeugen, die die eine oder mehrere Wellenlängen aufweist, die aus dem Bereich von 190-380 nm ausgewählt sind, wobei das 3D-Objekt (1) stromabwärts der Lichtquelle (10) konfiguriert ist.

## Revendications

1. Procédé de production d'un article en 3D (1) au moyen d'une modélisation de dépôt par fusion, le procédé comprenant une étape d'impression en 3D, dans lequel l'étape d'impression en 3D comprend une étape de dépôt de matériau réfléchissant, dans lequel l'étape de dépôt de matériau réfléchissant comprend :
- la fourniture d'un matériau imprimable en 3D (201) comprenant (i) un matériau de matrice polymère (211) qui est transmissif vis-à-vis du rayonnement UV, dans lequel le matériau de matrice polymère (211) comprend un matériau thermoplastique, et (ii) un matériau réfléchissant (212) qui est réfléchissant vis-à-vis du rayonnement UV et qui est au moins partiellement entouré par le matériau de matrice polymère (211) ; dans lequel le matériau réfléchissant (212) comprend un premier fluoropolymère ; et
- le dépôt du matériau imprimable en 3D (201), pour fournir l'article en 3D (1) comprenant le matériau imprimé en 3D (202) comprenant le matériau de matrice (211) et le matériau réfléchissant (212) ;
**caractérisé en ce que** le premier fluoropolymère comprend un premier polytétrafluoroéthylène microporeux.

2. Procédé selon la revendication 1, dans lequel le premier fluoropolymère comprend un fluoropolymère microporeux, ayant une porosité choisie dans la plage d'environ 35 à 45 %.

3. Procédé selon l'une quelconque des revendications précédentes, dans lequel le premier fluoropolymère comprend un premier fluoropolymère amorphe.

4. Procédé selon l'une quelconque des revendications précédentes, dans lequel le premier fluoropolymère comprend une première température de fusion de fluoropolymère T_{f1,m}, dans lequel le matériau de matrice (211) comprend un second fluoropolymère ayant une seconde température de fusion de fluoropolymère T_{f2,m} et/ou une seconde température de transition vitreuse de fluoropolymère T_{f2,g}, dans lequel T_{f2,m}≤T_{f1,m}-10 °C et/ou dans lequel T_{f2,g}≤T_{f1,m}-10 °C.

5. Procédé selon l'une quelconque des revendications 1 à 4 précédentes, dans lequel l'étape de dépôt de matériau réfléchissant comprend (a) le guidage d'une fibre (240) sans fusion à travers une buse d'imprimante 3D (502), tout en (b) fournissant le matériau de matrice polymère (211) à la buse d'imprimante 3D (502) pour fournir un matériau imprimé en 3D noyau-coque (202).

6. Procédé selon l'une quelconque des revendications 1 à 4 précédentes, dans lequel le matériau imprimable en 3D (201) comprend un matériau particulaire (250) comprenant le matériau réfléchissant (212), dans lequel le matériau particulaire (250) est incorporé dans le matériau de matrice (211).

7. Procédé selon la revendication 6, dans lequel le matériau particulaire (250) comprend un second matériau réfléchissant (250) choisi dans le groupe des particules de BaSO₄, des particules de TiO₂, des particules d'Al₂O₃, des particules d'argent, des particules d'aluminium et des paillettes réfléchissantes.

8. Procédé selon l'une quelconque des revendications 5 à 7 précédentes, comprenant le dépôt d'un matériau imprimable en 3D (201) comprenant un noyau (260) et une coque (270), dans lequel le noyau (260) comprend le matériau réfléchissant (212), dans lequel le noyau (260) comprend un matériau thermoplastique, et dans lequel la coque (270) comprend le matériau de matrice (211) ; dans lequel une couche (322) du matériau imprimé en 3D (202) a une largeur (w1) et une hauteur (h1), sélectionnées individuellement dans la plage de 0,1-10 mm ; dans lequel la coque (270) a la plus grande largeur de coque (wₛₘ₁), dans lequel la plus grande largeur de coque (wₛₘ₁) est choisie dans la plage de 2-15 % de la largeur (w1).

9. Procédé selon l'une quelconque des revendications précédentes, dans lequel l'article en 3D (1) comprend un réflecteur creux.

10. Article en 3D (1) comprenant un matériau imprimé en 3D (202), dans lequel l'article en 3D (1) comprend une pluralité de couches (322) de matériau imprimé en 3D (202), dans lequel au moins une partie de la pluralité de couches (322) comprend un matériau imprimé en 3D (202) comprenant (i) un matériau de matrice polymère (211) qui est transmissif vis-à-vis du rayonnement UV, et (ii) un matériau réfléchissant (212) qui est réfléchissant vis-à-vis du rayonnement UV et qui est au moins partiellement entouré par le matériau de matrice polymère (211) ; dans lequel le matériau réfléchissant (212) comprend un premier fluoropolymère, **caractérisé en ce que** le premier fluoropolymère comprend un premier polytétrafluoroéthylène microporeux.

11. Article en 3D (1) selon la revendication 10, dans lequel au moins une partie de la pluralité de couches (322) comprend un matériau imprimé en 3D noyau-coque (202), dans lequel le matériau imprimé en 3D noyau-coque (202) comprend un noyau (260) et une coque (270), dans lequel le noyau (260) comprend une fibre (240) comprenant le matériau réfléchissant (212), et dans lequel la coque (270) comprend le matériau de matrice polymère (211).

12. Article en 3D (1) selon l'une quelconque des revendications 10 ou 11 précédentes, dans lequel le matériau imprimé en 3D (202) comprend un matériau particulaire (250) comprenant le matériau réfléchissant (212), dans lequel le matériau particulaire (250) est noyé dans le matériau de matrice (211) ; dans lequel le matériau particulaire (250) comprend un second matériau réfléchissant (250) choisi dans le groupe des particules BaSO₄, des particules TiO₂, des particules Al₂O₃, des particules d'argent, des particules d'aluminium et des paillettes réfléchissantes ; dans lequel le matériau imprimé en 3D (201) comprend un noyau (260) et une coque (270), dans lequel le noyau (260) comprend le matériau réfléchissant (212) et dans lequel la coque (270) comprend le matériau de matrice (211) ; et dans lequel une ou plusieurs de la pluralité de couches (322) du matériau imprimé en 3D (202) ont une largeur (w1) et une hauteur (h1), choisies individuellement dans la plage de 0,1-10 mm ; dans lequel la coque (270) a la plus grande largeur de coque (wₛₘ₁), dans lequel la plus grande largeur de coque (w_{wsm1}) est choisie dans la plage de 2 à 15 % de la largeur (w1).

13. Dispositif d'éclairage (1000) comprenant l'article en 3D (1) selon l'une quelconque des revendications 10 à 12 précédentes, dans lequel l'article en 3D (1) est conçu comme un ou plusieurs des éléments suivants : (i) au moins une partie du boîtier du dispositif d'éclairage, (ii) au moins une partie d'une paroi d'une chambre d'éclairage, et (iii) un élément optique, dans lequel le dispositif d'éclairage (1000) comprend une source lumineuse (10) configurée pour générer un rayonnement UV (11) ayant une ou plusieurs longueurs d'onde choisies dans la plage de 190 à 380 nm, dans lequel l'article en 3D (1) est conçu en aval de la source lumineuse (10).
